# EUROPÄISCHE PATENTSCHRIFT

(11) **EP 3 863 705 B1**
(45) Veröffentlichungstag und Bekanntmachung des Hinweises auf die Patenterteilung: **20.07.2022**
(21) Anmeldenummer: 20725155.4
(22) Anmeldetag: 08.05.2020
(51) Int. Cl.: A61M 16/08, A61B 5/097

(54) **VERBINDUNGS-ANORDNUNG ZUM HERSTELLEN EINER FLUIDVERBINDUNG ZWISCHEN EINEM MEDIZINISCHEN GERÄT UND EINER PATIENTENSEITIGEN KOPPLUNGSEINHEIT**
CONNECTION ASSEMBLY FOR ESTABLISHING A FLUIDIC CONNECTION BETWEEN A MEDICAL DEVICE AND A PATIENT-SIDE COUPLING UNIT
ENSEMBLE DE CONNEXION POUR L'ÉTABLISSEMENT D'UNE COMMUNICATION FLUIDIQUE ENTRE UN APPAREIL MÉDICAL ET UNE UNITÉ DE COUPLAGE CÔTÉ PATIENT

(30) Priorität: 14.05.2019 DE 102019003395
(43) Veröffentlichungstag der Anmeldung: 18.08.2021
(73) Patentinhaber: Drägerwerk AG & Co. KGaA, 23558 Lübeck (DE)
(72) Erfinder: DUNKEL, Thorsten, 23558 Lübeck (DE)
(86) Internationale Anmeldenummer: PCT/EP2020/062839
(87) Internationale Veröffentlichungsnummer: WO 2020/229339

(56) Entgegenhaltungen:
- WO-A1-2014/097068
- CN-A- 102 274 567
- DE-A1- 19 949 283
- US-A- 4 838 258
- US-A- 5 101 834
- US-A- 5 284 160
- US-A- 5 722 391

## Beschreibung

Die Erfindung betrifft eine Anordnung, mit deren Hilfe sich eine Fluidverbindung zwischen einem medizinischen Gerät und einer patientenseitigen Kopplungseinheit herstellen lässt. Das medizinische Gerät umfasst beispielsweise ein Beatmungsgerät, welches einen Patienten künstlich beatmet oder dessen natürliche Atmung unterstützt, oder ein Anästhesiegerät, welches einen Patienten narkotisiert. Die Kopplungseinheit umfasst z.B. einen Tracheostoma-Katheter oder einen Endotrachealtubus.

In US 5,284,160 wird eine Verbindungs-Anordnung beschrieben, welche ein medizinisches Gerät (conventional gas machine 215) mit einem patientenseitigen Verbindungsstück (patient end connector 14) zu verbinden vermag. Mittig durch einen Koaxialschlauch mit einem äußeren Schlauch (flexible outer breathing hose 27) und einem inneren Schlauch (flexible inner breathing hose 29) hindurch ist ein Fluidmessschlauch (sampling hose 23, 201) hindurch geführt. Ein Adapter (sampling adaptor 12) ist mit dem patientenseitigen Verbindungsstück 14 verbunden und umfasst eine Halterung (apertured support 22) mit Naben 43 und einer mittigen Aussparung, durch welche hindurch der Fluidmessschlauch 23, 201 geführt ist.

In US 5,722,391 wird ein Koaxialschlauch für ein Anästhesiegerät (anesthesia tube assembly) mit einem äußeren Schlauch (outer tube 12) und einem inneren Schlauch (inner tube 14) beschrieben. Der patientenseitige Anschluss 20 der Anordnung umfasst einen äußeren Zylinder 21, der mit dem äußeren Schlauch 12 gasdicht verbunden ist, sowie einen inneren Zylinder 25, der mit dem inneren Schlauch 14 verbunden ist. Eine Leitung (extension pipe 40) vermag an einem Gasentnahmepunkt (gas sampling port 42) Gas zu entnehmen. Ein Gasmessschlauch (gas sampling tube 38) führt durch das Innere des inneren Schlauchs 14 und wird mit einer nicht gezeigten Klammer (clip) am inneren Zylinder 14 in der Nähe des inneren Zylinders 25 befestigt.

In DE 698 15 464 T2 wird eine Maske 1 zur Atmungsunterstützung beschrieben, welche auf der dem Gesicht 3 des Patienten gegenüberliegenden Seite eine rohrförmige Vorrichtung 5 umfasst. Diese Vorrichtung 5 hat einen inneren Durchgang 7 mit einer konischen Wand 8. Zwischen dieser Wand 8 und einer Ringkammer 12 werden Kanäle 9 um den konischen Teil 8 herum gebildet.

In US 4,838,258 wird eine Schlauch-Anordnung (breathing hose assembly 14) für ein Anästhesiegerät (anesthetic gas machine 18) beschrieben. Die Y-förmige Schlauch-Anordnung 14 umfasst zwei parallele Schläuche 16 und 17 sowie ein Y-Stück 13, welches mit einer Atemmaske (patient mask 11) verbunden ist. Durch den Schlauch 17 hindurch ist ein Gasmess-Schlauch (small sampling tube 21) mit einem patientenseitigen Eingang (sampling entrance end 22) geführt, der an seinem Ausgang mit einem Messgerät (gas sampling monitor 19) verbunden ist.

WO 2014 / 097068 A1 zeigt einen Beatmungskreislauf (patient circuit 20) zur Narkotisierung eines Patienten. Ein Beatmungs-Schlauch (main delivery conduit 22) verbindet einen Druckerzeuger (pressure generating device 12) mit einer patientenseitigen Koppeleinheit (patient interface device 40). Parallel zum Schlauch 22 ist ein Druckmess-Schlauch (main pressure feedback conduit 32) geführt. Sowohl der Beatmungs-Schlauch 22 als auch der Druckmess-Schlauch 32 erstrecken sich zwischen zwei Anschlussstücke (rotary coupling devices 60A, 60B).

CN 102274567 A zeigt ein Steuerventil (breathing tube control valve) für einen Beatmungsschlauch (gas pipeline). Das Steuerventil umfasst ein Gehäuse 1, einen rohrförmigen Einlass (air inlet tube 11), einen rohrförmigen Auslass (air outlet tube 12) und ein inneres Rohr (inner tube 100). Ein Blockierelement (partition 110) kann den Einlass 11 versperren. Ein Kolben (piston assembly 4) kann das Blockierelement 110 bewegen.

In US 5,101,834 wird ein Y-förmiger Adapter 12 beschrieben, der zwei Schenkel (legs 13 and 14) sowie ein Mittelstück (center leg 15) umfasst. Der Schenkel 14 ist mit einem Schlauch 18 zum Zuführen von Gas zu einem Patienten verbunden, der Schenkel 13 mit einem Schlauch 17 zum Abführen von Gas und das Mittelstück 15 mit einer Atemmaske (face mask 11). Ein Gasmess-Schlauch (sampling tube 27) ist in einem Verbindungsstück (coupler 29) mit einem Schlauch (tube 31) verbunden, und der Schlauch 31 führt zu einem Gasmessgerät (gas analyzer 32). Der Gasmess-Schlauch 27 wird beweglich im Mittelstück 15 gehalten. Am Einlassende (sampling end 28) des Gasmess-Schlauchs 27 ist eine Verbreiterung (radially enlarged portion 33) vorgesehen, welche verhindert, dass der Gasmess-Schlauch 27 aus dem Adapter 12 herausgezogen wird.

DE 199 49 283 A1 zeigt einen Atemschlauch 1 mit einer Anschlussvorrichtung 2 umfassend einen Basiskörper 3 mit zwei Kopplungsabschnitten 4 und 5. Im Inneren des Atemschlauchs 1 ist ein Druckmess-Schlauch 8 geführt, der über einen Durchführungskanal 9 im Basiskörper 3 in den Kopplungsabschnitt 5 mündet. Der Druckmess-Schlauch 8 führt in einem Absatz 10 am Ende des Durchführungskanals 9.

Die in EP 2 383 008 B1 beschriebene Vorrichtung hält ein Volumen von Atemgas, mit dem ein Lebewesen (subject 8) beatmet wird, auf einem gewünschten Niveau. Ein Beatmungsgerät (ventilator 2) versorgt das Lebewesen 8 über einen ersten Schlauch (first tubing 6) und erhält ausgeatmetes Gas über einen zweiten Schlauch (second tubing 7). Die beiden Schläuche 6, 7 können parallel nebeneinander angeordnet sein oder als ein Koaxialschlauch ausgestaltet sein. Ein Adapter 4 ist mit den beiden Schläuchen 6, 7 verbunden und zweigt eine Gasprobe an einem Verbindungselement (sample output connector 10) ab. Ein Gasmessschlauch (sampling tube 11) führt von dem Verbindungselement 10 zu einem Analysiergerät (gas analyzer 12) am Beatmungsgerät 2.

In WO 2017/200929 A1 werden ein Verfahren und eine Vorrichtung beschrieben, um mittels Seitenstrom-Kapnographie den Gehalt von Kohlendioxid in der Luft, die ein Patient 201, 301 ausatmet, zu messen. Der Patient ist an ein Beatmungsgerät 250, 350 angeschlossen. Ein Adapter 220, 320 zweigt Atemluft in einen Schlauch 215, 315 ab, der zu einem Messgerät 210, 310 führt.

Auch in EP 1 551 486 B1 und in US 2015 / 0 343 163 A1 werden Vorrichtungen beschrieben, um zu untersuchendes Gas aus einem Atemluft-Kreislauf für einen Patienten abzuzweigen und einem Messgerät zuzuführen.

In US 2014 / 0 018 691 A1 wird eine Anordnung zur künstlichen Beatmung eines Patienten beschrieben. Eine Sauerstoffquelle 135 versorgt über einen Schlauch 136 den Patienten mit Sauerstoff, wobei eine Atemmaske 125 über das Gesicht des Patienten gelegt ist. Vom Patienten ausgeatmete Luft wird über einen Schlauch 132 einem Gasmessgerät 130 zugeführt.

Der Erfindung liegt die Aufgabe zugrunde, eine Verbindungs-Anordnung zum Herstellen einer Fluidverbindung zwischen einer medizinischen Anordnung und einer patientenseitigen Kopplungseinheit bereitzustellen, wobei die Verbindungs-Anordnung eine zuverlässigere Überwachung eines Fluidstroms in der hergestellten Fluidverbindung als bekannte Verbindungs-Anordnungen ermöglicht.

Die erfindungsgemäße Verbindungs-Anordnung ist dafür ausgestaltet, eine Fluidverbindung zwischen einem medizinischen Gerät und einer patientenseitigen Kopplungseinheit herzustellen. Die patientenseitige Kopplungseinheit lässt sich mit einem Patienten koppeln oder anderweitig am Patienten anlegen.

Die erfindungsgemäße Verbindungs-Anordnung umfasst
- einen Versorgungsschlauch,
- ein patientenseitiges Verbindungsstück,
- einen Fluidmessschlauch und
- einen Adapter.

Der Versorgungsschlauch ist mit dem medizinischen Gerät verbunden oder lässt sich mit dem medizinischen Gerät verbinden. Der Versorgungsschlauch ist darüber hinaus mit dem patientenseitigen Verbindungsstück verbunden.

Gesehen in eine Fließrichtung vom medizinischen Gerät zur patientenseitigen Kopplungseinheit befindet das patientenseitige Verbindungsstück sich am Ende des Versorgungsschlauchs oder ist mit einem Abstand flussabwärts vom Versorgungsschlauch positioniert. Der Versorgungsschlauch und das patientenseitige Verbindungsstück stellen wenigstens zeitweise zusammen mindestens eine Fluidverbindung zwischen dem medizinischen Gerät und der patientenseitigen Kopplungseinheit her oder vermögen mindestens eine solche Fluidverbindung herzustellen. Mithilfe dieser Fluidverbindung lässt sich der Patient mit Fluid, insbesondere Atemluft, versorgen.

Der Adapter umfasst
- eine starre Adapter-Röhre und
- eine Adapter-Röhrenhalterung.

Das patientenseitige Verbindungsstück hält die Adapter-Röhrenhalterung. Die Adapter-Röhrenhalterung umgibt vollständig oder wenigstens teilweise die Adapter-Röhre.

Die Adapter-Röhre hält einen patientenseitigen Endabschnitt des Fluidmessschlauchs. Die Adapter-Röhrenhalterung hält dadurch den Endabschnitt des Fluidmessschlauchs, den die Adapter-Röhre hält, in einer vorgegebenen Position relativ zum patientenseitigen Verbindungsstück, in einer Ausgestaltung am patientenseitigen Ende des Verbindungsstücks. Die Adapter-Röhre ist starr, während der Fluidmessschlauch biegsam ist, genauer: eine größere Plastizität als die Adapter-Röhre aufweist.

Die Verbindungs-Anordnung stellt eine Mess-Fluidverbindung zwischen einem Fluidmessgerät und einer vorgegebenen Fluidentnahmeposition her oder vermag wenigstens zeitweise eine solche Mess-Fluidverbindung herzustellen. Diese Fluidentnahmeposition befindet sich im Inneren des patientenseitigen Verbindungsstücks. Die bereitgestellte Mess-Fluidverbindung umfasst den Fluidmessschlauch.

Das medizinische Gerät soll in einer typischen Anwendung den Patienten mit der richtigen Menge eines Gases versorgen, beispielsweise mit Atemluft und / oder einem Anästhesiegas. Bei einer Abweichung zwischen einer geforderten Menge, beispielsweise einer geforderten Fließrate, und einer tatsächlichen Menge, z.B. der tatsächlichen Fließrate, soll das medizinische Gerät entsprechend angesteuert werden, damit es seine Arbeitsweise mit dem Ziel ändert, die geforderte Menge bereitzustellen. In einer anderen Anwendung soll das medizinische Gerät die spontane Atmung des Patienten automatisch überwachen. Um eine derartige Arbeitsweise zu ermöglichen, ist es erforderlich, die tatsächliche Menge des Gases sicher zu messen, beispielsweise die Menge von zugeführter oder spontan eingeatmeter Atemluft oder die Menge oder der Anteil von Kohlendioxid in der vom Patienten P ausgeatmeten Luft oder der Anteil eines Anästhesiegases im zugeführten Gas. Die Messung muss verlässlich und reproduzierbar sein. Das Fluidmessgerät vermag die Konzentration mindestens eines Gases zu messen und steht in einer Datenverbindung mit dem medizinischen Gerät.

Erfindungsgemäß ist zusätzlich zu dem Versorgungsschlauch, mit dessen Hilfe sich eine Fluidverbindung, z.B. ein Fluidkreislauf, zwischen dem Patienten und dem medizinischen Gerät herstellen lässt, ein Fluidmessschlauch vorgesehen. Dank dieses Fluidmessschlauchs lässt sich permanent oder auch bei Bedarf eine bestimmte zu prüfende Menge von Gas aus dem Inneren der Verbindungs-Anordnung absaugen und dem Fluidmessgerät zuführen, wobei dieses Fluidmessgerät in einer Datenverbindung mit dem medizinischen Gerät steht und sich außerhalb der Verbindungs-Anordnung befindet.

Der Fluidmessschlauch ermöglicht es, die zu prüfende Menge an der vorgegebenen Fluidentnahmeposition zu entnehmen, insbesondere abzusaugen. Erfindungsgemäß befindet diese Fluidentnahmeposition sich im Inneren des patientenseitigen Verbindungsstücks. Gesehen in eine Fließrichtung von medizinischen Gerät zum Patienten befindet sich die Fluidentnahmeposition sich somit am Ende des oder flussabwärts vom Versorgungsschlauch und relativ nahe zum Patienten. Zwischen einer patientenseitigen Koppeleinheit und der Fluidentnahmeposition befindet sich somit ein relativ kleines umschlossenes Volumen, in welchem sich Fluid ansammeln könnte, welches eine Messung verfälschen könnte. Die erfindungsgemäße Ausgestaltung, wo sich die Fluidentnahmeposition befindet, ist insbesondere dann wichtig, wenn der Versorgungsschlauch mindestens zwei parallele Lumen enthält, insbesondere eine für die Inspiration (Einatmung) und eine weitere für die Exspiration (Ausatmen), und daher einen Fluidkreislauf ermöglicht. Die Fluidentnahmeposition lässt sich zwischen diesen beiden Lumen und der patientenseitigen Kopplungseinheit anordnen. Somit lässt sich die gesuchte Messung sowohl in einer Inspirationsphase als auch in einer Exspirationsphase des Patienten zuverlässig durchführen.

Zu berücksichtigen ist die Möglichkeit, dass sich die Verbindungs-Anordnung und damit die Fluidentnahmeposition relativ zum Patienten bewegen. Beispielsweise bewegt der Patient sich oder wird bewegt, oder die Verbindungs-Anordnung ist einer sonstigen von außen wirkenden Kraft ausgesetzt. Außerdem fließt häufig durch eine Verbindungs-Anordnung permanent ein sogenannter Spülflow oder Kreisflow, der ebenfalls eine Kraft auf den Fluidmessschlauch ausübt. Auch in dieser Situation sollte die Fluidentnahmeposition möglichst keine Bewegung relativ zum patientenseitigen Verbindungsstück und damit auch keine Bewegung relativ zur patientenseitigen Kopplungseinheit ausführen, weil eine solche Relativbewegung die Messergebnisse verfälschen könnte.

Erfindungsgemäß wird der Endabschnitt des Fluidmessschlauchs von der Adapter-Röhre gehalten, und zwar in einer vorgegebenen Position relativ zum patientenseitigen Verbindungsstück. Dadurch wird sichergestellt, dass die Fluidentnahmeposition im Wesentlichen keine unerwünschte Relativbewegung relativ zum patientenseitigen Verbindungsstück ausführt.

Erfindungsgemäß wird der patientenseitige Endabschnitt mithilfe einer Röhre gehalten, also flächig entlang eines Abschnitts des Fluidmessschlauchs. Erfindungsgemäß befindet sich das patientenseitige Ende der starren Adapter-Röhre an der Fluidentnahmeposition. Diese Merkmale verringern die Gefahr einer unerwünschten Relativbewegung verglichen mit einer möglichen Ausgestaltung, bei der der biegsame Fluidmessschlauch lose im Versorgungsschlauch oder im patientenseitigen Verbindungsstück liegt oder nur an einem Punkt gehalten wird.

Insbesondere sind die beiden folgenden alternativen Ausführungsformen möglich, um die Erfindung zu realisieren:
1. An der Fluidentnahmeposition befindet sich das patientenseitige Ende der Adapter-Röhre. Das patientenseitige Ende des Fluidmessschlauchs weist einen Abstand zu dieser Fluidentnahmeposition auf. Die Adapter-Röhre überbrückt diesen Abstand, weist eine fluiddichte Mantelfläche auf und ist in Fluidkommunikation mit dem Fluidmessschlauch.
2. Das patientenseitige Ende des Fluidmessschlauchs befindet sich an der Fluidentnahmeposition und wird dort von der Adapter-Röhre gehalten. Auch in dieser Ausführungsform befindet sich das patientenseitige Ende der Adapter-Röhre an der Fluidentnahmeposition.

Gemäß der ersten Alternative verlängert also die starre Adapter-Röhre den biegsamen Fluidmessschlauch zur Fluidentnahmeposition hin. Die Adapter-Röhre kann so ausgestaltet sein, dass sie sich sicher mit der Adapter-Röhrenhalterung und damit mit dem patientenseitigen Verbindungsstück verbinden lässt, beispielsweise indem sie aus dem gleichen Werkstoff hergestellt ist, der bevorzugt ein steifer Werkstoff ist, beispielsweise aus einem starren Kunststoff. Möglich ist, dass das patientenseitige Verbindungsstück und der gesamte Adapter zusammen ein einziges Bauteil bilden. Der Fluidmessschlauch lässt sich hingegen aus einem flexiblen Werkstoff, beispielsweise PVC oder Silikon oder Gummi, fertigen. Das patientenseitige Ende des Fluidmessschlauchs lässt sich mit der Adapter-Röhre in einem Bereich verbinden, der einen Abstand zu der Fluidentnahmeposition aufweist. Die Verbindung zwischen dem Fluidmessschlauch und der Adapter-Röhre beeinflusst daher weniger die Fluidentnahmeposition, als wenn die Verbindung an der Fluidentnahmeposition angeordnet wäre.

Gemäß der zweiten Alternative hat die Adapter-Röhre im Wesentlichen die Funktion, den Fluidmessschlauch zu halten und eine unerwünschte Relativbewegung zu vermeiden. Dank der Adapter-Röhre bleibt das patientenseitige Ende des Fluidmessschlauchs an der Fluidentnahmeposition. Die Adapter-Röhre braucht bei der zweiten Alternative nicht notwendigerweise fluiddicht ausgestaltet zu sein, was die Herstellung vereinfacht. Weil auch in dieser Ausgestaltung das patientenseitige Ende der Adapter-Röhre sich an der Fluidentnahmeposition befindet, wird die Gefahr einer unerwünschten Relativbewegung verglichen mit einer anderen Positionierung der Adapter-Röhre verringert.

Erfindungsgemäß vermag die Verbindungs-Anordnung eine Mess-Fluidverbindung zwischen dem medizinischen Gerät und einer vorgegebenen Fluidentnahmeposition herzustellen. Bevorzugt verbindet diese Mess-Fluidverbindung die Fluidentnahmeposition mit einem Fluidmessgerät, welches in einer Datenverbindung mit dem medizinischen Gerät steht.

In einer Ausgestaltung umgibt das patientenseitige Verbindungsstück die Adapter-Röhrenhalterung und damit auch die Adapter-Röhre. In einer Ausgestaltung ist gesehen in eine Fließrichtung vom medizinischen Gerät zur patientenseitige Kopplungseinheit die Abmessung desjenigen Teils der Adapter-Röhre, welche sich in der Adapter-Röhrenhalterung befindet, mindestens halb so groß, bevorzugt mindestens zwei Drittel so groß wie die Abmessung des patientenseitigen Verbindungsstücks in diese Fließrichtung. Dadurch überbrückt die Adapter-Röhre wenigstens die Hälfte des patientenseitigen Verbindungsstücks.

In einer Ausgestaltung umgibt das patientenseitige Verbindungsstück die Adapter-Röhrenhalterung fluiddicht. In einer anderen Ausgestaltung umgibt die Adapter-Röhrenhalterung das patientenseitige Verbindungsstück fluiddicht. Beide Ausgestaltungen reduzieren weiter die Gefahr, dass Fluid von außen in die Mess-Fluidverbindung eindringt und die Messergebnisse, die mithilfe des Fluidmessschlauchs gewonnen werden, verfälscht.

Erfindungsgemäß hält die Adapter-Röhre den patientenseitigen Endabschnitt des Fluidmessschlauchs. In einer Ausgestaltung umgibt die Adapter-Röhre den gehaltenen Endabschnitt fluiddicht. In einer anderen Ausgestaltung umgibt der gehaltene Endabschnitt des Fluidmessschlauchs die Adapter-Röhre fluiddicht. Beide Ausgestaltungen lassen sich insbesondere in Kombination mit der oben beschriebenen ersten Ausführungsform realisieren, bei welcher die Adapter-Röhre einen Abstand zwischen der Fluidentnahmeposition und dem patientenseitigen Ende des Fluidmessschlauchs überbrückt. Die Ausgestaltung, dass die Adapter-Röhre den Endabschnitt fluiddicht umgibt oder umgekehrt, führt zu einer besonders sicheren Halterung des Fluidmessschlauchs und reduziert die Gefahr, dass Fluid von außen eindringt und die Messungen verfälscht.

In einer Ausgestaltung ist ein Endstück des Versorgungsschlauchs mit der Adapter-Röhrenhalterung fluiddicht verbunden. Auch diese Ausgestaltung reduziert die Gefahr, dass Messungen verfälscht werden. Einerseits könnte ohne eine solche fluiddichte Verbindung Gas aus dem Versorgungsschlauch austreten, die Adapter-Röhrenhalterung umgehen und die Fluidentnahmeposition erreichen. Andererseits könnte Fluid von außen in den Versorgungsschlauch eindringen.

Vorzugsweise ist die Adapter-Röhre vollständig oder wenigstens teilweise im Inneren des patientenseitigen Verbindungsstücks angeordnet. Dadurch umgibt das patientenseitige Verbindungsstück wenigstens teilweise die Adapter-Röhre und schützt die Adapter-Röhre vor mechanischen Belastungen, die von außen einwirken. Die Adapter-Röhre braucht daher nicht dicker als erforderlich ausgestaltet zu sein, um den Fluidmessschlauch zu halten und die betriebssichere Fluidentnahme zu ermöglichen.

In einer Ausgestaltung ist der patientenseitige Endabschnitt des Fluidmessschlauchs im Inneren der Adapter-Röhre eingeklemmt. Oder die Adapterröhre ist im Inneren des Endabschnitts eingeklemmt. Die Adapter-Röhre wiederum ist fest mit der Adapter-Röhrenhalterung verbunden. Diese Ausgestaltung hält den patientenseitigen Endabschnitt des Fluidmessschlauchs besonders sicher und verhindert, dass der Fluidmessschlauch eine unerwünschte Bewegung relativ zur Fluidentnahmeposition vollführt. In einer bevorzugten Realisierung dieser Ausgestaltung, um den patientenseitigen Endabschnitt zu halten, ist der patientenseitige Endabschnitt im Inneren der Adapter-Röhre eingeklemmt. Der Innendurchmesser der Adapter-Röhre lässt sich gegen eine Rückstellkraft vergrößern, beispielsweise aufgrund einer Elastizität und / oder Plastizität der Adapter-Röhre. Die Rückstellkraft klemmt den patientenseitigen Endabschnitt im Inneren der Adapter-Röhre ein.

Erfindungsgemäß befindet sich die vorgegebene Fluidentnahmeposition im Inneren des patientenseitigen Verbindungsstücks oder im Inneren des Adapters. In einer Ausgestaltung befindet die Fluidentnahmeposition sich im Inneren des patientenseitigen Verbindungsstücks und mit einem Abstand zum patientenseitigen Ende dieses Verbindungsstücks oder an diesem patientenseitigen Ende. In der Regel ist das patientenseitige Verbindungsstück größer und oft auch mechanisch robuster als der Adapter, so dass es von Vorteil ist, die Fluidentnahmeposition mit Bezug auf das patientenseitige Verbindungsstück festzulegen.

Bevorzugt befindet die Fluidentnahmeposition sich auf einer gedachten Mittelachse des Versorgungsschlauchs oder einer gedachten Verlängerung dieser Mittelachse auf den Patienten zu. Unterschiedliche Positionen, wie die Adapter-Röhre relativ zum Versorgungsschlauch angeordnet ist, sind möglich.

Bevorzugt setzt sich die Mittelachse des patientenseitigen Verbindungsstücks in der Mittelachse des Versorgungsschlauchs fort. In einer Ausgestaltung sind der Fluidmessschlauch und die Adapter-Röhre relativ zum Versorgungsschlauch und damit auch relativ zum patientenseitigen Verbindungsstück zentriert angeordnet.

In einer alternativen Ausgestaltung tritt ein Abstand zwischen der Mittelachse des Versorgungsschlauchs und der Mittelachse der Adapter-Röhre und / oder der Mittelachse des Fluidmessschlauchs auf. In einer Fortbildung dieser alternativen Ausgestaltung steht die Adapter-Röhre in Fluidkommunikation mit einer Verbindungsröhre. Diese Verbindungsröhre ist bevorzugt in Fluidkommunikation mit dem Fluidmessschlauch. Die Verbindungsröhre ist senkrecht oder schräg zur Mittelachse des Versorgungsschlauchs positioniert. Diese Fortbildung lässt sich mit der ersten Ausführungsform kombinieren, bei welcher die Adapter-Röhre einen Abstand zwischen der Fluidentnahmeposition und dem patientenseitigen Endabschnitt des Fluidmessschlauchs überbrückt. Der Fluidmessschlauch wird mithilfe der Adapter-Röhre und der Verbindungsröhre bis zur Fluidentnahmeposition hin verlängert, die zentriert angeordnet sein kann, also insbesondere auf eine Mittelachse des patientenseitigen Verbindungsstücks.

Bevorzugt hält das verbindungsseitige Verbindungsstück den Adapter. In einer Ausgestaltung ist der Adapter fest mit dem patientenseitigen Verbindungsstück verbunden. Diese Ausgestaltung erhöht die mechanische Stabilität und die Funktionssicherheit der Verbindungs-Anordnung. In einer Fortbildung dieser Ausgestaltung sind das patientenseitige Verbindungsstück und der Adapter einstückig ausgebildet, bilden also ein einziges Bauteil. Dies erhöht weiter die mechanische Stabilität. Besonders bevorzugt sind das patientenseitige Verbindungsstück und der Adapter sogar monolithisch ausgestaltet, d. h. sie werden beim Herstellen der Verbindungs-Anordnung in einem einzigen Prozessschritt gefertigt, beispielsweise durch Spritzgießen. Diese Ausgestaltung reduziert die Herstellkosten und / oder die benötigte Herstellzeit im Vergleich zu anderen möglichen Ausgestaltungen.

Bevorzugt ist die Adapter-Röhre fest mit dem patientenseitigen Endabschnitt des Fluidmessschlauchs verbunden. Bei dieser Ausgestaltung hält die Adapter-Röhre einen kompletten Endabschnitt des Fluidmessschlauchs, was die Gefahr einer unerwünschten Relativbewegung des Fluidmessschlauchs verglichen mit einer nur punktförmigen Halterung reduziert. Besonders bevorzugt wird die Verbindung durch eine Klebeverbindung oder eine Schweißverbindung hergestellt.

Der Fluidmessschlauch kann sich außerhalb des Verbindungsschlauchs befinden. In einer bevorzugten Ausgestaltung befindet sich der Fluidmessschlauch hingegen im Inneren des Versorgungsschlauchs und ist durch den Versorgungsschlauch hindurch geführt. In dieser Ausgestaltung umgibt der Versorgungsschlauch den Fluidmessschlauch, was Platz einspart und die Gefahr reduziert, dass der Fluidmessschlauch mechanisch beschädigt wird oder aus der gewünschten Position herausgerissen wird.

Der Versorgungsschlauch kann in einer Ausgestaltung nur eine einzige Fluidverbindung realisieren. In einer bevorzugten Ausgestaltung ist der Verbindungsschlauch hingegen als ein Mehr-Lumen-Schlauch realisiert, d.h. mindestens zwei Fluidverbindungen lassen sich gleichzeitig mithilfe desselben Versorgungsschlauchs herstellen, z.B. in unterschiedliche Richtungen und / oder für verschiedene Fluide. Die mindestens zwei Lumen des Versorgungsschlauchs sind fluiddicht voneinander getrennt. Beispielsweise fließt der Fluidstrom zur Beatmung oder Narkotisierung des Patienten (Inspirations-Fluss) durch das eine Lumen und der Fluidstrom, der beim Ausatmen des Patienten auftritt (Exspirations-Fluss), durch das oder ein anderes Lumen. Die Ausgestaltung als Mehr-Lumen-Schlauch spart Platz ein im Vergleich zu einer möglichen Ausgestaltung mit mehreren parallelen Versorgungsschläuchen. Bevorzugt ist der Fluidmessschlauch durch eines dieser Lumen geführt. Er kann insbesondere durch das Inspirations-Lumen oder das Exspirations-Lumen geführt sein. Möglich ist auch, dass ein drittes Lumen den Fluidmessschlauch realisiert oder aufnimmt.

Verschiedene Ausgestaltungen sind möglich, um die erfindungsgemäße Verbindungs-Anordnung herzustellen. In einer Ausgestaltung wird die erfindungsgemäße Verbindungs-Anordnung durch ein Verfahren hergestellt, welches die folgenden Schritte umfasst:
- Der Fluidmessschlauch wird von einer Seite in den Versorgungsschlauch hineingeschoben. Der Fluidmessschlauch wird soweit hineingeschoben, bis ein patientenseitiger Endabschnitt des Fluidmessschlauchs über den Versorgungsschlauch übersteht.
- Von der anderen Seite wird der Adapter auf den Versorgungsschlauch zu bewegt. Bei dieser Bewegung wird der überstehende patientenseitige Endabschnitt in die Adapter-Röhre eingeschoben.
- Nunmehr wird der Adapter mit dem patientenseitigen Verbindungsstück verbunden.
- Außerdem wird der Adapter mit dem Versorgungsschlauch verbunden.

Diese Ausgestaltung ermöglicht es, verschiedene Bestandteile der Verbindungs-Anordnung getrennt voneinander herzustellen und erst an einem Einsatzort zusammenzusetzen.

Im Folgenden wird die Erfindung anhand eines Ausführungsbeispiels beschrieben. Hierbei zeigen:
- Figur 1: schematisch eine Verbindungs-Anordnung zwischen einem medizinischen Gerät und einem Patienten;
- Figur 2: in perspektivischer Darstellung die Verbindungs-Anordnung;
- Figur 3: drei mögliche Verläufe eines Gasmessschlauchs, welcher Gas zu einem Gasmessgerät führt;
- Figur 4: vier nachteilhafte und eine bevorzugte Messposition, an welcher Gas in den Gasmessschlauch von Figur 3 geführt wird;
- Figur 5: eine mögliche Positionen eines Adapters zwischen dem Koaxialschlauch und dem Y-Stück;
- Figur 6: eine erste Ausgestaltung einer ersten Ausführungsform des Adapters, hält den Gasmessschlauch im inneren Lumen;
- Figur 7: eine zweite Ausgestaltung der ersten Ausführungsform des Adapters, hält den Gasmessschlauch im äußeren Lumen;
- Figur 8: eine mögliche Realisierung der zweiten Ausgestaltung, bei welcher der Adapter und das Y-Stück zusammen ein einziges Bauteil bilden;
- Figur 9: eine dritte Ausgestaltung der ersten Ausführungsform des Adapters, hält den Gasmessschlauch außerhalb des Koaxialschlauchs;
- Figur 10: eine erste Ausgestaltung einer zweiten Ausführungsform des Adapters, hält den Gasmessschlauch im inneren Lumen;
- Figur 11: eine zweite Ausgestaltung der zweiten Ausführungsform des Adapters, hält den Gasmessschlauch im äußeren Lumen;
- Figur 12: eine dritte Ausgestaltung der zweiten Ausführungsform des Adapters, hält den Gasmessschlauch außerhalb des Koaxialschlauchs.

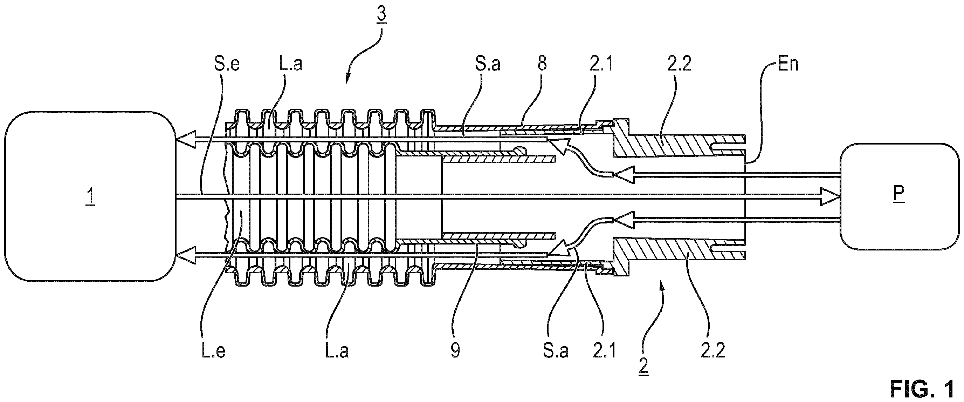

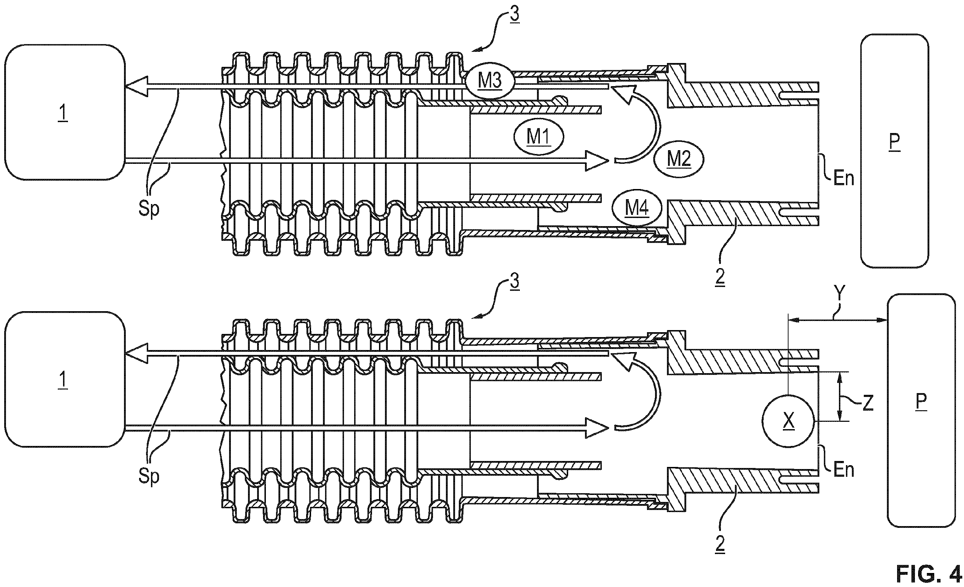

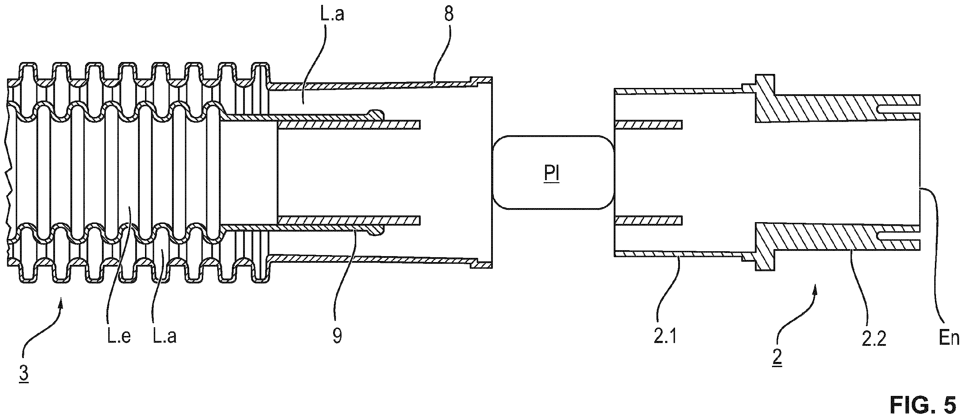

Figur 1 bis Figur 3 zeigen ein beispielhaftes Umfeld, in welchem die Erfindung eingesetzt wird. Figur 4 veranschaulicht einen durch die Erfindung erzielbaren Vorteil, und Figur 5 bis Figur 12 zeigen zwei bevorzugte Ausführungsformen des Ausführungsbeispiels.

Im Ausführungsbeispiel wird die Erfindung in einer Verbindungs-Anordnung eingesetzt, welche einen Patienten mit einem medizinischen Gerät verbindet - genauer gesagt: einen Fluidkreislauf zwischen dem Patienten und dem medizinischen Gerät herstellt. In einer Ausgestaltung umfasst das medizinische Gerät ein Beatmungsgerät. Das Beatmungsgerät steuert insbesondere die Atemphasen des Patienten und / oder unterstützt und / oder stimuliert seine Atmung. In einer anderen Ausgestaltung umfasst das medizinische Gerät ein Anästhesiegerät, mit dem ein Patient narkotisiert werden kann.

Die Verbindungs-Anordnung umfasst
- einen patientenseitigen Anschluss, der mit einer in Figur 1 nicht gezeigten patientenseitigen Koppeleinheit verbunden oder verbindbar ist, wobei die patientenseitige Koppeleinheit im Ausführungsbeispiel ein L-förmiges Anschlussstück und ein Mundstück umfasst,
- einen geräteseitigen Anschluss, der mit dem medizinischen Gerät verbunden oder verbindbar ist und z.B. ein T-Stück oder ein Endstück umfasst, und
- einen Mehr-Lumen-Schlauch.

In einer Ausgestaltung lässt sich die Verbindungs-Anordnung mehrfach verwenden. In einer anderen Ausgestaltung werden die gesamte Verbindungs-Anordnung sowie die patientenseitige Koppeleinheit vorab zu einem System verbunden, und dieses System wird in einer geeigneten Umhüllung zu einem Einsatzort transportiert, dort aus der Umhüllung entnommen, mit der patientenseitigen Koppeleinheit und mit dem medizinischen Gerät verbunden, einmal zum Herstellen einer Fluidverbindung verwendet und anschließend entsorgt.

Unter einen Mehr-Lumen-Schlauch wird ein Schlauch verstanden, der mindestens zwei Lumen über die gesamte Länge des Schlauchs bereitstellt, wobei jedes Lumen von dem oder jedem anderen Lumen fluiddicht getrennt ist. Dadurch können durch denselben Schlauch gleichzeitig verschiedene Fluidströme fließen, beispielsweise in zwei unterschiedliche Richtungen und / oder mit unterschiedlichen Fluiden und / oder mit unterschiedlichen Fließraten. Insbesondere lässt sich ein Beatmungskreislauf für einen Patienten mit einem einzigen Versorgungsschlauch realisieren, falls dieser als ein Mehr-Lumen-Schlauch ausgebildet ist. Im Ausführungsbeispiel ist der Mehr-Lumen-Schlauch als ein Koaxialschlauch ausgestaltet. Möglich ist auch, dass eine Membrane den Schlauch entlang der gesamten Länge in mindestens zwei Lumen unterteilt, wobei jedes Lumen von innen an eine Außenwand des Schlauchs und an die Membrane angrenzt.

Der Koaxialschlauch des Ausführungsbeispiels umfasst
- einen inneren Schlauch, der ein inneres Lumen bildet, sowie
- einen äußeren Schlauch, der ein äußeres Lumen bildet.

Der äußere Schlauch umgibt im Ausführungsbeispiel koaxial und vollständig den inneren Schlauch. Im Ausführungsbeispiel wird der innere Schlauch mit dem inneren Lumen für das Einatmen (Inspiration) verwendet, der äußere Schlauch mit dem äußeren Lumen für das Ausatmen (Exspiration). Möglich ist auch, die beiden Lumen anders herum zu verwenden oder zwei parallele Schläuche zu verwenden. Die beiden Schläuche sind fluiddicht voneinander getrennt.

Figur 1 zeigt schematisch diese Verbindungs-Anordnung zwischen dem Patienten und dem medizinischen Gerät umfassend das Beatmungsgerät und/oder das Anästhesiegerät. Dargestellt werden:
- der Patient P,
- das medizinische Gerät 1,
- ein flexibler Koaxialschlauch 3,
- das vom inneren Schlauch des Koaxialschlauchs 3 gebildete innere Lumen L.e,
- das vom äußeren Schlauch des Koaxialschlauchs 3 gebildete äußere Lumen L.a,
- ein starres oder biegsames inneres Endstück 9 des inneren Schlauchs,
- ein starres oder biegsames äußeres Endstück 8 des äußeren Schlauchs, welches das innere Endstück 9 vollständig und koaxial umgibt und über das innere Endstück 8 übersteht,
- ein Y-Stück 2, welches mit der patientenseitigen Koppeleinheit verbunden oder verbindbar ist und ein patientenseitiges Ende En aufweist,
- der Luftstrom S.e beim Einatmen, der durch das innere Lumen L.e fließt, und
- der Luftstrom S.a beim Ausatmen, der durch das äußere Lumen L.a fließt.

Das Y-Stück 2 ist auf einer Seite sowohl mit dem inneren als auch mit dem äußeren Schlauch verbunden und nimmt sowohl das innere Lumen L.e als auch das äußere Lumen L.a auf. Auf der anderen Seite verbindet das Y-Stück 2 beide Lumen L.e und L.a mit einem Verbindungsstück zur der patientenseitigen Koppeleinheit. Daher wird die Bezeichnung Y-Stück verwendet. Die Stücke 8 und 9 auf der einen Seite und 2 auf der anderen Seite sind fluiddicht miteinander verbunden, und zwar fest oder lösbar. Im Ausführungsbeispiel umfasst das Y-Stück 2 ein Teil 2.1 mit einem größeren Durchmesser, welches mit dem Koaxialschlauch 3 verbunden ist, und ein axial anschließendes Teil 2.2 mit einem kleineren Durchmesser, welches mit dem Verbindungsstück verbunden ist. Beispielsweise lässt sich das röhrenförmige Verbindungsstück in entsprechende Ausnehmungen des kleineren Teils 2.2 einstecken. Das Y-Stück 2 gehört im Ausführungsbeispiel zum patientenseitigen Verbindungsstück der Verbindungs-Anordnung.

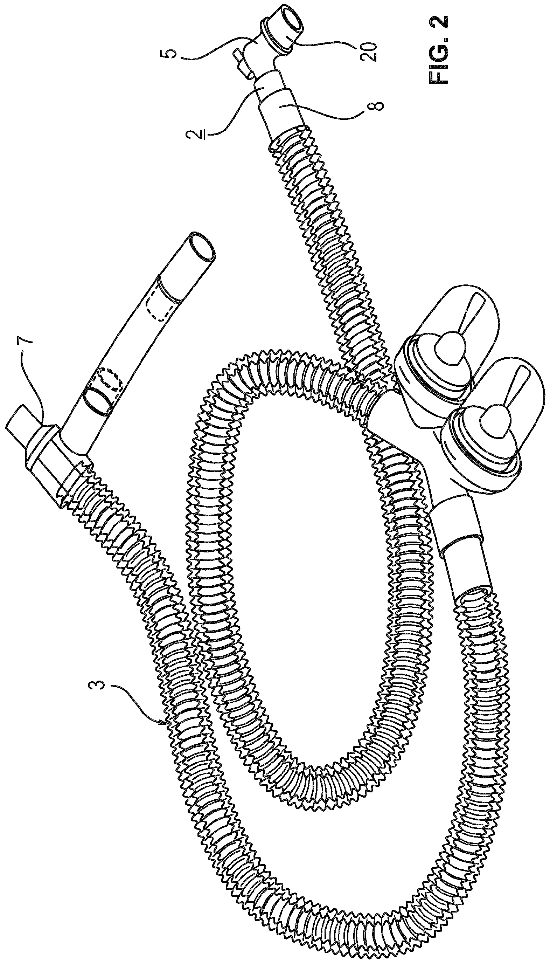

Figur 2 zeigt in perspektivischer Darstellung eine beispielhafte Ausführung eines Verbindungs-Systems umfassend die Verbindungs-Anordnung und die patientenseitige Kopplungseinheit. Gezeigt werden
- der Koaxialschlauchs 3 mit dem äußeren Endstück 8,
- das Y-Stück 2 und
- ein L-förmiges Anschlussstück 5, welches zur patientenseitigen Kopplungseinheit gehört und in das Teil 2.2 eingesteckt oder vom Teil 2.2 umgeben ist.

Das Anschlussstück 5 ist fluiddicht mit dem Y-Stück 2 verbunden, und zwar fest oder lösbar. Dieser Koaxialschlauch 3 ist biegsam und lässt sich in beinahe jede gewünschte Form bringen. Weiterhin werden ein Mundstück 20, welches zu der patientenseitigen Kopplungseinheit gehört, und ein T-Stück 7, welches zum geräteseitigen Anschluss gehört, gezeigt.

Während der Patient P beatmet und / oder narkotisiert wird, soll ein Gas untersucht werden, welches im Zusammenhang mit der Beatmung und / oder Narkotisierung des Patienten P steht. Beispielsweise soll der Anteil von Kohlendioxid oder die Konzentration eines Anästhesiemittels in der dem Patienten P zugeführten Luft und / oder in der vom Patienten P ausgeatmeten Luft untersucht werden. Bei dieser Untersuchung müssen über einen längeren Zeitraum hinweg gleichartige äußere Bedingungen vorliegen, insbesondere um den aktuellen Zustand des Patienten P sicher beurteilen zu können.

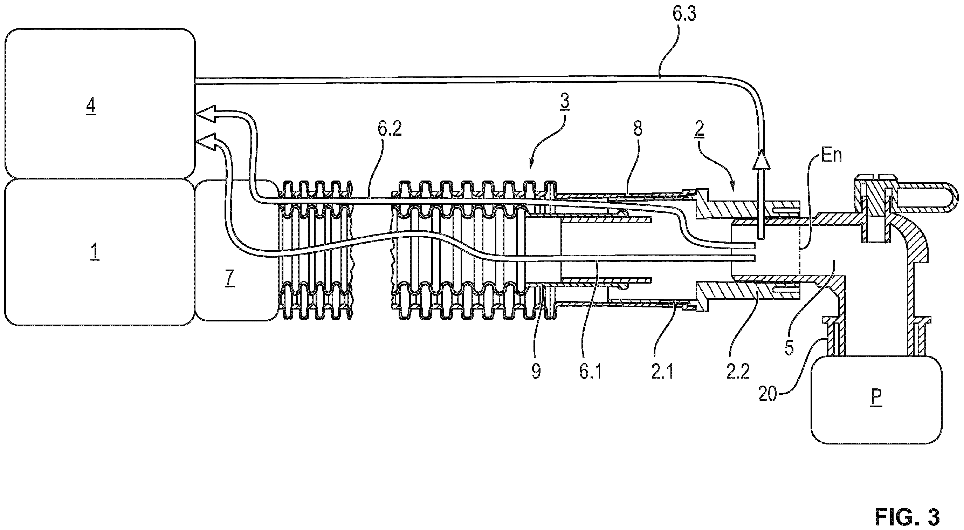

Figur 3 zeigt mehrere Möglichkeiten, wie ein solches Gerät 4 zur Untersuchung von zugeführtem oder abgeführtem Gas mit dem Patienten P verbunden wird. Gas fließt durch einen Gasmessschlauch 6 zu einem Gasmessgerät 4. Gezeigt werden drei mögliche Positionen, wie ein Gasmessschlauch 6 verlegt sein kann, nämlich
- ein Gasmessschlauch 6 mit inspiratorischen Verlauf 6.1 im inneren Lumen L.e,
- ein Gasmessschlauch 6 mit exspiratorischen Verlauf 6.2 im äußeren Lumen L.a und
- ein Gasmessschlauch 6 mit externem Verlauf 6.3, also außerhalb des Koaxialschlauches 3.

Ein Problem, das beim Einsatz einer Verbindungs-Anordnung mit einem Gasmessschlauch 6 auftreten kann, resultiert aus einer Krümmung, welche dem Gasmessschlauch 6 durch die Herstellung und den Transport zu einem Einsatzort aufgeprägt ist. In der Regel wird eine Rolle mit einem langen Schlauch hergestellt. Ein Segment von diesem langen aufgerollten Schlauch wird abgeschnitten und als Gasmessschlauch 6 verwendet. Die Erfindung ermöglicht es, einen Gasmessschlauch 6 trotz der ihm aufgeprägten Krümmung in einem Verlauf zu halten, der aus diesen drei möglichen Verläufen 6.1, 6.2, 6.3 ausgewählt ist. Die Position, an welcher zu untersuchendes Gas in den Gasmessschlauch 6 eintritt, relativ zum Y-Stück 2 bleibt dank der Erfindung trotz der aufgeprägten Krümmung unverändert, auch wenn die Verbindungs-Anordnung bewegt wird.

Das T-Stück 7 zwischen dem Koaxialschlauch 3 und dem medizinischen Gerät 1 ermöglicht es, einen Gasmessschlauch 6 mit dem Verlauf 6.1 oder 6.2 im Inneren des Koaxialschlauchs 3 aus dem Koaxialschlauch 3 herauszuführen. Zu untersuchendes Gas tritt an dem patientenseitigen Ende in den Gasmessschlauch 6 ein.

Während der Patient P beatmet und / oder narkotisiert wird, fließt ein Fluss von Gas von der medizinischen Anordnung 1 durch den Koaxialschlauch 3 bis zum patientenseitigen Anschluss und wieder zurück. Dieser Gasfluss wird Spülflow oder Kreisflow genannt, verhindert einen Gasstau in der Verbindungs-Anordnung und hat vorzugsweise eine konstante Volumenflussrate. Figur 4 zeigt diesen Spülflow Sp. Weiterhin zeigt Figur 4 im oberen Bild vier mögliche Messpositionen M1 bis M4, die unterschiedliche Nachteile aufweisen, und im unteren Bild eine bevorzugte Messposition X, welche durch die Erfindung betriebssicher erzielt werden kann. Diese insgesamt fünf Messpositionen haben folgende Bedeutung:
- Bei der Messposition M1 endet der Gasmessschlauch 6 im inneren Lumen L.e. Dadurch vermag das Gerät 4 nur frisches Atemgas zu untersuchen, welches im Luftstrom S.e von dem medizinischen Gerät 1 zum Patienten P fließt, aber kein exspiriertes Atemgas im Luftstrom S.a.
- Bei der Messposition M2 endet der Gasmessschlauch 6 im unmittelbaren Übergang zwischen Inspiration und Exspiration. Diese Position hat den Nachteil, dass der Spülflow Sp die Messergebnisse verfälschen kann.
- Bei der Messposition M3 endet der Gasmessschlauch 6 im äußeren Lumen L.a. Das Gerät 4 vermag dadurch nur exspiriertes Atemgas im Luftstrom S.a zu entdecken, aber kein frisches Atemgas im Luftstrom S.e.
- Bei der Messposition M4 endet der Gasmessschlauch 6 in einem Randbereich des Y-Stücks 2. Die Gefahr besteht, dass der Gasmessschlauch 6 teilweise oder sogar ganz verschlossen wird und eine valide Messung nicht gewährleistet werden kann.
- Eine ideale Messposition X liegt außerhalb des Spülflows Sp und mit einem konstanten Abstand y zum Patienten P und mit einem konstanten Abstand z zur Außenwand des Y-Stücks 2.

Im Folgenden werden mit Bezug auf Figur 5 bis Figur 12 zwei Ausführungsformen der Erfindung beschrieben. Die erste Ausführungsform umfasst einen Adapter 10 mit einer integrierten Röhre, in welche sich ein Gasmessschlauch 6 einführen lässt. Die zweite Ausführungsform umfasst einen Adapter 11, welcher den Gasmessschlauch 6 im Inneren aufnimmt und hält. In beiden Ausführungsformen hält der Adapter 10, 11 einen patientenseitigen Endabschnitt EA des Gasmessschlauchs 6 so, dass zu untersuchendes Gas an der gewünschten Messposition X aufgenommen wird. Bei beiden Ausführungsformen befindet das patientenseitige Ende der Adapter-Röhre sich an der Messposition X. Bei der ersten Ausführungsform tritt ein Abstand zwischen dem patientenseitigen Endabschnitt EA und der Messposition X auf, den die Adapter-Röhre überbrückt. Bei der zweiten Ausführungsform befindet sich zusätzlich das patientenseitige Ende des Gasmessschlauchs 6 an der Messposition X.

Figur 5 zeigt, an welchem Platz Pl ein solcher Adapter 10, 11 für den Gasmessschlauch 6 angebracht werden kann. In beiden Ausgestaltungen lässt sich eine Röhre oder Hülse in das Innere des Adapters 10, 11 stecken, so dass mindestens eine Fluidverbindung durch den Adapter 10, 11 verlaufen kann. Möglich ist, dass dieser Adapter 10, 11 und das Y-Stück 2 als ein einzelnes Bauteil ausgeführt sind.

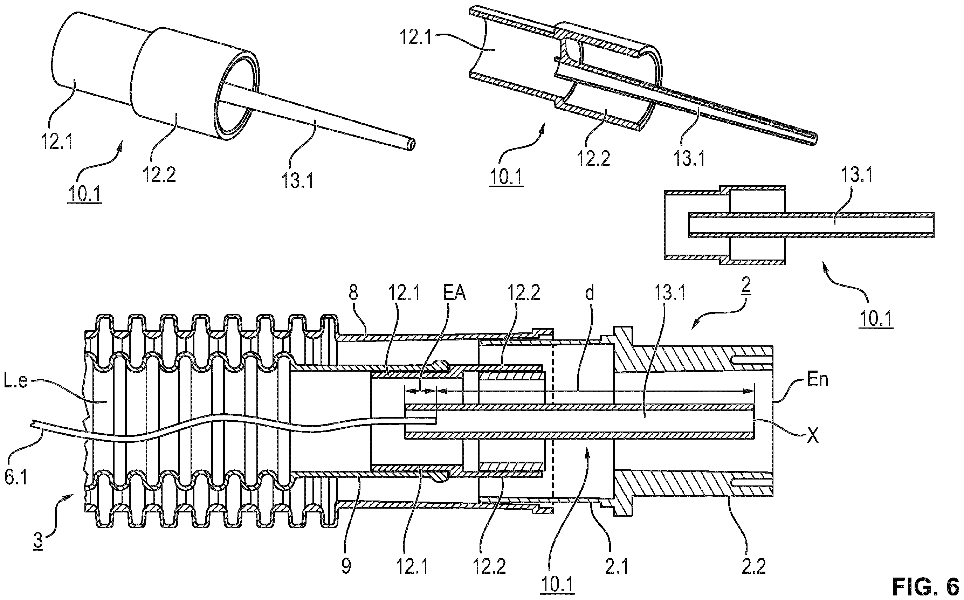

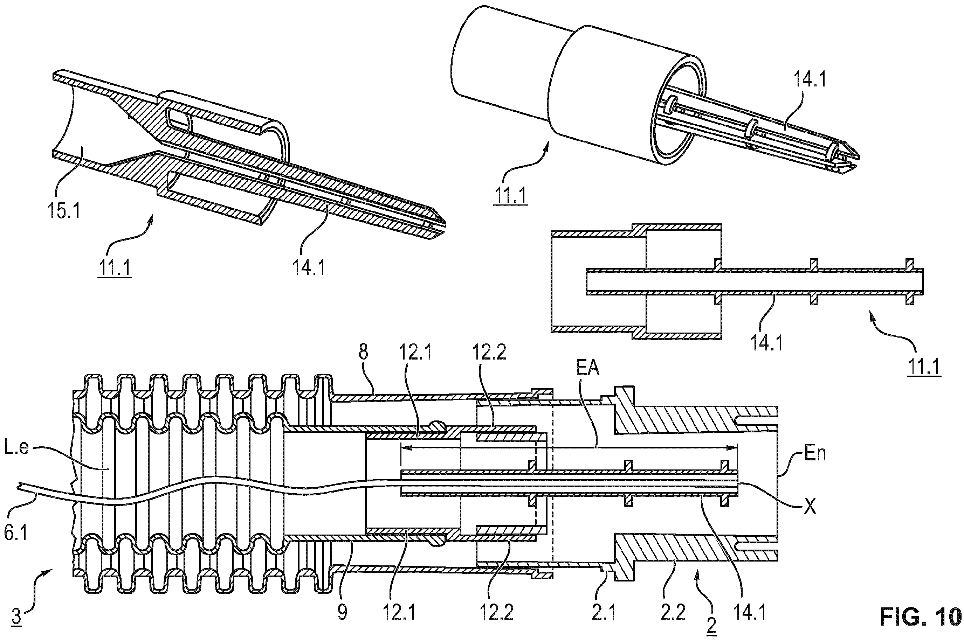

Figur 6 bis Figur 9 zeigen drei mögliche Ausgestaltungen der ersten Ausführungsform, nämlich drei unterschiedlich ausgestaltete Adapter 10.1, 10.2, 10.3. Ein Adapter 10.1, 10.2, 10.3 gemäß der ersten Ausführungsform kann auch eine Kombination von zwei oder allen drei Ausgestaltungen aufweisen. Figur 10 bis Figur 12 zeigen drei mögliche Ausgestaltungen der zweiten Ausführungsform.

In den Figuren ist die jeweils erzielte ideale Messposition X eingezeichnet.

Der Adapter 10.1 gemäß der ersten Ausgestaltung, die in Figur 6 gezeigt wird, vermag einen Gasmessschlauch 6 zu halten, welcher im inneren Lumen L.e des Koaxialschlauchs 3 angeordnet ist, also den inspiratorischen Verlauf 6.1 aufweist.

Der Adapter 10.1 umfasst
- eine erste Hülse 12.1 mit einem kleineren Durchmesser,
- eine zweite Hülse 12.2 mit einem größeren Durchmesser, die fest mit der ersten Hülse 12.1 verbunden ist, sowie
- eine Röhre 13.1, die durch beide Hülsen 12.1, 12.2 hindurch geführt ist.

In der gezeigten Ausgestaltung steht die Röhre 13.1 zum Y-Stück 2 hin über beide Hülsen 12.1, 12.2 über. Möglich ist auch, dass die Röhre 13.1 bündig mit der Hülse 12.2 abschließt oder im Inneren der Hülse 12.1 oder 12.2 endet. Bevorzugt ist der Adapter 10.1 so ausgestaltet, dass die Mittelachsen der beiden Hülsen 12.1 und 12.2 und der Röhre 13.1 übereinstimmen.

Der Außendurchmesser der kleineren Hülse 12.1 ist in einer Ausgestaltung gleich dem Innendurchmesser des inneren Endstücks 9. In einer anderen Ausgestaltung ist der Außendurchmesser der Hülse 12.1 etwas größer als der Innendurchmesser des Endstücks 9, und zwar wenigstens an dem Ende, das an die Hülse 12.2 angrenzt. Falls die Hülse 12.1 in das Endstück 9 eingeschoben wird, so wird die Hülse 12.1 zusammengedrückt, oder das Endstück 9 wird gedehnt.

In allen Realisierungen wird die innere Hülse 12.1 fluiddicht von dem inneren Endstück 9 aufgenommen. Der Außendurchmesser der größeren Hülse 12.2 ist gleich dem Innendurchmesser des zum Schlauch 3 hin zeigenden Segments des Y-Stücks 2 oder etwas größer, sodass die zweite Hülse 12.2 fluiddicht vom Y-Stück 2 aufgenommen wird. Bevorzugt ist der Außendurchmesser der zweiten Hülse 12.2 etwa gleich dem Außendurchmesser des inneren Endstücks 9, sodass die zweite Hülse 12.2 fluchtend mit dem inneren Endstück 9 abschließt.

In einer Ausgestaltung ist der Adapter 10.1 fest mit dem Y-Stück 2 verbunden. Möglich ist, dass der Adapter 10.1 und das Y-Stück 2 einstückig ausgebildet sind. Möglich ist sogar, dass die beiden Bestandteile 10.1 und 2 monolithisch ausgestaltet sind, also bei der Herstellung in einem einzigen Prozessschritt und aus dem gleichen Werkstoff hergestellt werden.

Möglich ist auch, dass der Adapter 10.1 lösbar oder nicht lösbar mit dem Y-Stück 2 verbunden ist. In dieser alternativen Realisierung lässt sich ein bereits vorhandenes Y-Stück 2 mit einem neuartigen Adapter 10.1 verbinden. Möglich, aber dank der vorteilhaften Ausgestaltung der Erfindung nicht erforderlich ist es, das Y-Stück 2 abzuändern.

Bei der Ausgestaltung, die in Figur 6 gezeigt wird, stehen die Röhre 13.1 und damit der Gasmessschlauch 6 in der Position 6.1 in Fluidkommunikation mit dem Luftstrom beim Einatmen S.e, der durch das innere Lumen L.e fließt. Daher erreicht ein Teil des Luftstroms S.e den Gasmessschlauch 6. Ein Endstück des Gasmessschlauchs 6 ist in die Röhre 13.1 eingeschoben. Daher hält der Adapter 10.1 den Gasmessschlauch 6 in der inspiratorischen Position 6.1. Der Adapter 10.1 wird vom Y-Stück 2 gehalten und kann sich nicht relativ zum Y-Stück 2 bewegen. Das patientenseitige Ende der Röhre 13.1 befindet sich an der Messposition X. Die Röhre 13.1 überbrückt fluiddicht den Abstand zwischen der Messposition X und dem patientenseitigen Ende des Gasmessschlauchs 6.

Die Verbindung zwischen den Hülsen 12.1 und 12.2 einerseits und der Röhre 13.1 andererseits weist bevorzugt eine gewisse Elastizität auf, sodass der Abstand sich reversibel verändern kann und Energie absorbiert wird. Dies reduziert die Gefahr, dass ein mechanischer Impuls auf die Verbindungs-Anordnung den Gasmessschlauch 6 beschädigt oder zu einem Leck führt.

Die Röhre 13.1 umgibt bevorzugt fluiddicht einen patientenseitigen Endabschnitt des Gasmessschlauchs 6. Möglich ist auch, dass der Gasmessschlauch 6 ohne Überlappung fluiddicht mit der Röhre 13.1 verbunden ist.

In einer bevorzugten Ausgestaltung ist der Gasmessschlauch 6 fest oder lösbar mit der Röhre 13.1 verbunden, beispielsweise mithilfe einer Klebeverbindung oder einer geeigneten Schweißverbindung oder auch einer Klemmverbindung. Dank der Erfindung ist es aber ebenfalls möglich, dass der Gasmessschlauch 6 lediglich in die Röhre 13.1 eingeschoben wird und lösbar gehalten wird, z.B. von einer geeigneten Schnapphalterung. Dank der lösbaren Verbindung ist es leichter, die Verbindungs-Anordnung zu reinigen. Beide Ausgestaltungen verhindern sicher, dass der Gasmessschlauch 6 aus der Röhre 13.1 rutscht.

In beiden Ausgestaltungen wird die Verbindungs-Anordnung bevorzugt wie folgt zusammengesetzt:
- Der Gasmessschlauch 6 wird vom geräteseitigen Anschluss her durch das innere Lumen L.e zum patientenseitigen Anschluss hin geschoben, bis er ausreichend weit über das innere Endstück 9 übersteht.
- Der Adapter 10.1 wird von der anderen Seite auf den Gasmessschlauch 6 hin geschoben. In einer Ausgestaltung wird der Adapter 10.1 mitsamt dem Y-Stück 2 auf den Gasmessschlauch 6 hin geschoben.
- Bevorzugt wird ein Teil des überstehenden Endes des Gasmessschlauchs 6 fest oder lösbar mit dem Adapter 10.1 verbunden.
- Die erste Hülse 12.1 des Adapters 10.1 wird vollständig in das innere Endstück 9 eingeschoben und in einer Ausgestaltung mit diesem Endstück 9 verbunden.
- Der Gasmessschlauch 6 befindet sich nunmehr im inneren Lumen L.e und wird von der Röhre 13.1 im inspiratorischen Verlauf 6.1 gehalten.

In einer Ausgestaltung lässt der Adapter 10.1 sich wieder aus dem inneren Endstück 9 ziehen, beispielsweise um die Verbindungs-Anordnung zu reinigen, und erneut befestigen.

In der ersten Ausführungsform ist es nicht erforderlich, dass ein Ende des Gasmessschlauchs 6 an der gewünschten Position X, die in Figur 4 gezeigt wird, positioniert wird. Vielmehr reicht es aus, den Adapter 10.1 so auszugestalten, dass das patientenseitige Ende der Röhre 13.1 sich an der Messposition X befindet. Zwischen dem patientenseitigen Ende des Gasmessschlauchs 6 und der Messposition X tritt ein Abstand d auf, den die Röhre 13.1 überbrückt. Zu untersuchendes Gas strömt von der Messposition X durch die innen hohle Röhre 13.1 zu dem patientenseitigen Ende des Gasmessschlauchs 6 und durch diesen zu dem Gasmessgerät 4.

In der gezeigten Realisierung der ersten Ausgestaltung verjüngt sich die Röhre 13.1 gesehen in eine Richtung vom Koaxialschlauch 3 auf das Y-Stück 2 hin. Möglich ist auch, dass die Röhre 13.1 über die gesamte Länge den gleichen Durchmesser aufweist oder sich in die andere Richtung, also auf den Koaxialschlauch 3 zu, verjüngt. Möglich ist auch, dass die Röhre 13.1 konvex ausgestaltet ist und das patientenseitige Ende des Gasmessschlauchs 6 in einem mittigen Bereich der Röhre 13.1 einklemmt.

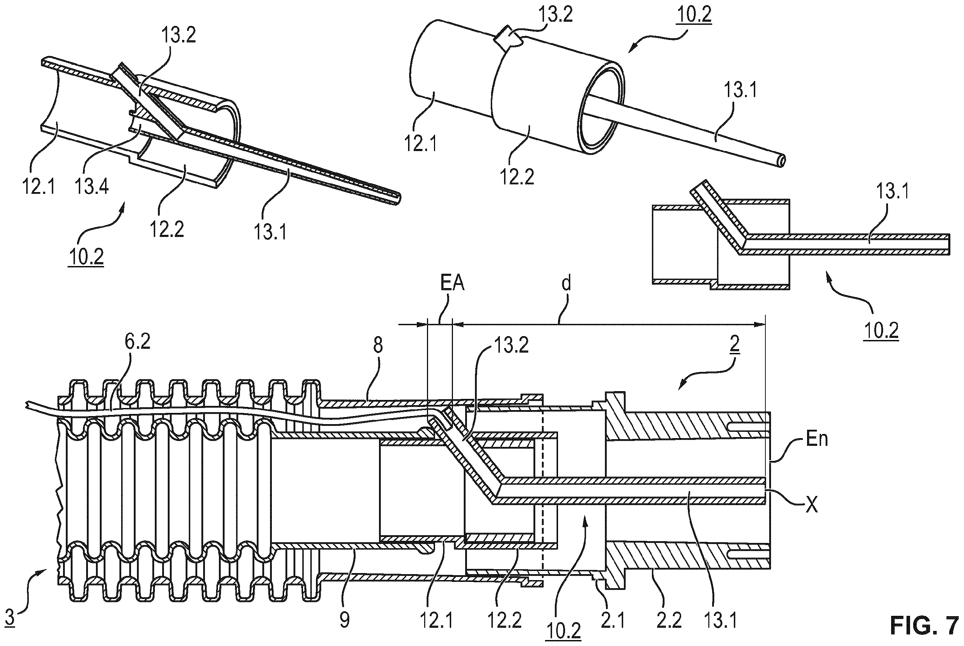

Figur 7 zeigt eine zweite Ausgestaltung der ersten Ausführungsform, nämlich den Adapter 10.2. In die koaxial angeordnete Röhre 13.1 ist in schräger oder senkrechter Position eine Verbindungsröhre 13.2 eingesetzt, die in der gezeigten Realisierung zylinderförmig ist. Die Verbindungsröhre 13.2 ist durch die zweite Hülse 12.2 hindurch geführt und verbindet die erste Röhre 13.1 mit einem Anschlusspunkt außerhalb des Adapters 10.2. Ein Gasmessschlauch 6 lässt sich durch das äußere Lumen L.a hindurch in die Verbindungsröhre 13.2 einführen und mit dieser fest oder lösbar verbinden. Der Adapter 10.2 hält dann den Gasmessschlauch 6 in einer Position, die einen exspiratorischen Verlauf 6.2 bewirkt. Bevorzugt ist der Gasmessschlauch 6 mit dem geräteseitigen Ende der Verbindungsröhre 13.2 fest verbunden.

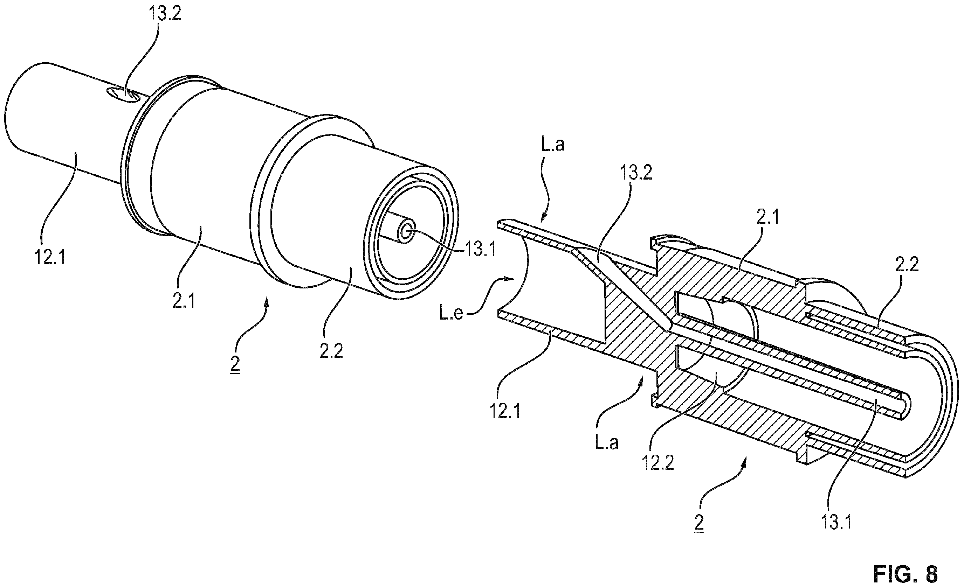

In einer Ausführungsform steht ein Segment 13.4 im Inneren der Hülse 12.1 und / oder 12.2 über die Verbindungsröhre 13.2 über, vgl. Figur 8 oben. Möglich ist auch, dieses Segment 13.4 fortzulassen und die mittige Röhre 13.1 und die Verbindungsröhre 13.2 zusammen als eine einstückige abgewinkelte Röhre auszugestalten, vgl. Figur 8 unten.

Die Verbindungs-Anordnung mit dem Adapter 10.2 lässt sich genauso zusammensetzen wie die Verbindungs-Anordnung mit dem Adapter 10.1. Wiederum wird das Gas an der Position X entnommen, ohne dass es erforderlich ist, den Gasmessschlauch 6 selber bis zu dieser Position X zu führen. Zu untersuchendes Gas strömt vielmehr von der Messposition X durch die Röhre 13.1, wird von der Verbindungsröhre 13.2 umgeleitet und fließt dann durch den Gasmessschlauch 6 zum Gasmessgerät 4.

Figur 8 zeigt in einer perspektivischen Darstellung (links) und einer Schnittdarstellung (rechts) eine beispielhafte Realisierung, in welcher der Adapter 10.2 der zweiten Ausgestaltung und das Y-Stück 2 einstückig ausgebildet sind. In diesem Beispiel schließt das patientenseitige Ende der Röhre 13.1 bündig mit dem patientenseitigen Ende des kleineren Teils 2.2 des Y-Stücks 2 ab. Die Röhren 13.1 und 13.2 bilden eine einstückige abgewinkelte Röhre.

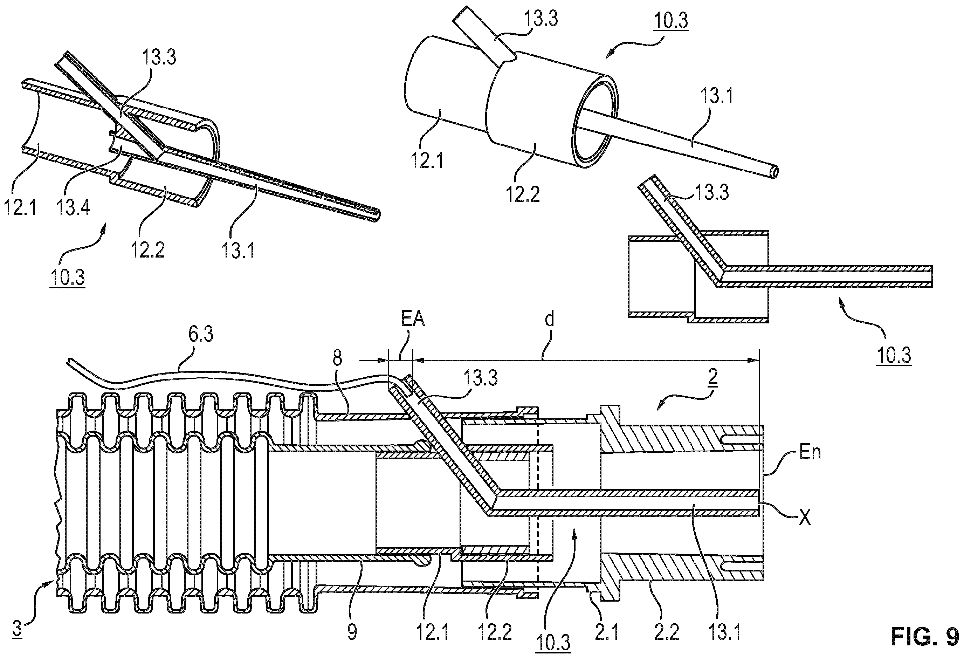

Figur 9 zeigt eine dritte Ausgestaltung der ersten Ausführungsform, nämlich den Adapter 10.3. Die Verbindungsröhre 13.2 gemäß der zweiten Ausgestaltung ist durch eine längere Verbindungsröhre 13.3 ersetzt. Diese längere Verbindungsröhre 13.2 ist ebenfalls schräg oder senkrecht durch die zweite Hülse 12.2 hindurch geführt und verbindet die erste Röhre 13.1 mit einem Anschlusspunkt außerhalb des äußeren Endstücks 8. Ein Gasmessschlauch 6 lässt sich außerhalb des Koaxialschlauchs 3 in die längere Verbindungsröhre 13.3 einführen. Der Adapter 10.2 hält dann den Gasmessschlauch 6 in einer Position, die einen externen Verlauf 6.3 bewirkt. Auch in der dritten Ausgestaltung ist es möglich, das Segment 13.4 fort zu lassen und die mittige Röhre 13.1 und die Verbindungsröhre 13.3 als einstückige abgewinkelte Röhre auszugestalten. Auch in der dritten Ausgestaltung wird Gas an der Position X entnommen, ohne dass es erforderlich ist, den Gasmessschlauch 6 selber an der gewünschten Position X zu halten.

Den drei Ausgestaltungen der ersten Ausführungsform ist gemeinsam, dass das Gas von der Position X durch eine innen hohle Röhre 13.1 und optional durch eine Verbindungsröhre 13.2, 13.3 zu dem Gasmessschlauch 6 strömt. Das patientenseitige Ende der Röhre 13.1 befindet sich stets an der Messposition X.

Die Adapter 11.1, 11.2, 11.3 der zweiten Ausführungsform umfassen anstelle einer innen hohlen Röhre 13.1 ein röhrenförmiges Führungselement 14.1, wobei der Gasmessschlauch 6 durch das Innere dieser Führungsröhre 14.1 bis hin zu der Messposition X geführt ist. Gleiche Teile haben übereinstimmende Bezugszeichen. Das patientenseitige Ende des Gasmessschlauchs 6 und bevorzugt auch das patientenseitige Ende der Führungsröhre 14.1 befinden sich also an der Messposition X. Ein patientenseitiges Segment des Gasmessschlauchs 6 ist mit dem Inneren der Führungsröhre 14.1 verbunden, beispielsweise durch Kleben oder Schweißen oder Klemmen oder durch eine Schnapphalterung. Möglich ist, dass die Führungsröhre 14.1 aus zwei miteinander verbundenen Halbröhren besteht, die ein patientenseitiges Ende des Gasmessschlauchs 6 zwischen sich einklemmen.

Die zweite Ausgestaltung ermöglicht es, dass der Gasmessschlauch 6 und die Führungsröhre 14.1 sich über eine längere Strecke überlappen als der Gasmessschlauch 6 und die Röhre 13.1 der ersten Ausgestaltung. Dadurch steht mehr Platz für eine dauerhafte oder lösbare Verbindung zur Verfügung. Die Führungsröhre 14.1 kompensiert eine dem Gasmessschlauch 6 aufgeprägte Krümmung.

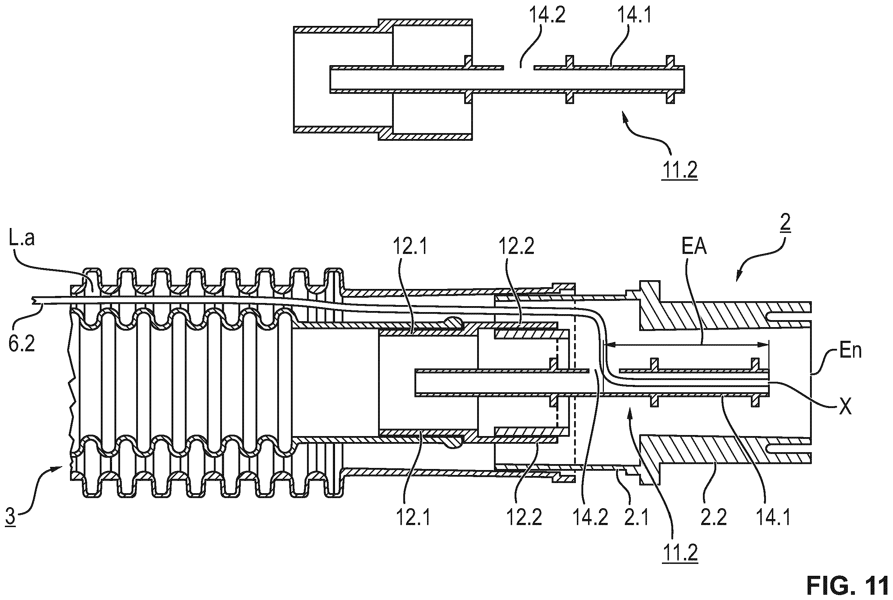

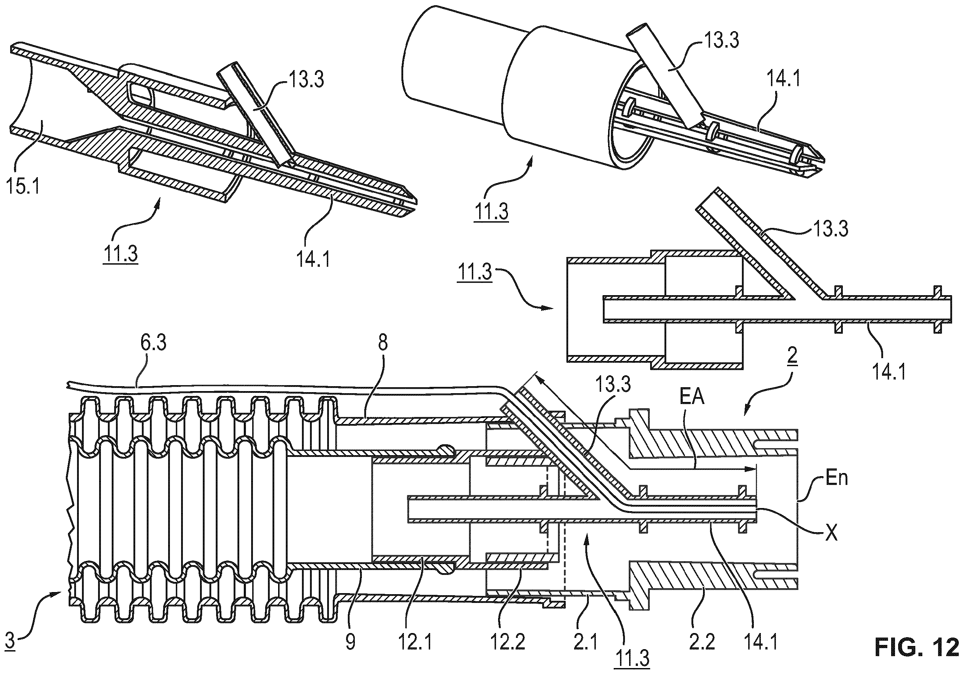

Der Adapter 11.1 bewirkt einen inspiratorischen Verlauf 6.1 des Gasmessschlauchs 6 (Figur 10), der Adapter 11.2 einen exspiratorischen Verlauf 6.2 (Figur 11) und der Adapter 11.3 einen externen Verlauf 6.3 (Figur 12). In allen drei Ausgestaltungen besitzt die Führungsröhre 14.1 bevorzugt mehrere ringförmige Verstärkungen, welche die Führungsröhre 14.1 umgeben. Die Führungsröhre 14.1 kann ein spitz zulaufendes patientenseitiges Ende aufweisen.

Der Adapter 11.1 und der Adapter 11.3 weisen eine abgewandelte erste Hülse mit einem sich zum patientenseitigen Ende hin kegelförmig verjüngenden Innenraum 15.1 auf. Die erste Hülse kann auch genauso wie die erste Hülse 12.1 des Adapters 10.1 aufgebaut sein.

In die Führungsröhre 14.1 des Adapters 11.2 ist eine Öffnung 14.2 eingelassen. Der Gasmessschlauch 6 ist durch diese Öffnung 14.2 hindurch geführt, um den exspiratorischen Verlauf 6.2 zu bewirken. Anstelle der Öffnung 14.2 kann auch eine Verbindungsröhre 13.2 wie beim Adapter 10.2 vorgesehen sein.

**BEZUGSZEICHENLISTE**

| | |
|---|---|
| 1 | medizinisches Gerät, umfasst im Ausführungsbeispiel ein Beatmungsgerät, welches die Atemphasen des Patienten P steuert und / oder dessen Atmung stimuliert, und / oder ein Anästhesiegerät, welches den Patienten P narkotisiert, sowie das Gasmessgerät 4 |
| 2 | Y-Stück, fluiddicht mit dem Endstück 9 und dem Verbindungsstück 5 verbunden, nimmt das äußere Lumen L.a und das innere Lumen L.e auf, ist mit dem Adapter 10, 11 verbunden, nimmt die Hülse 12.2 auf |
| 3 | Koaxialschlauch, gehört zur Verbindungs-Anordnung zwischen dem Patienten P und dem medizinischen Gerät 1, umfasst einen inneren und einen äußeren Schlauch |
| 4 | Gasmessgerät zur Untersuchung eines Gases, beispielsweise zur Ermittlung der Kohlendioxidkonzentration in der Atemluft oder der Konzentration an Anästhesiemittel, ist in einer Datenverbindung mit dem medizinischen Gerät 1 |
| 5 | L-förmiges Verbindungsstück zwischen dem Y-Stück 2 und dem Mundstück 20, gehört zur patientenseitigen Kopplungseinheit |
| 6 | Gasmessschlauch, verbindet das Untersuchungsgerät 4 mit dem Y-Stück 2 |
| 6.1 | inspiratorischer Verlauf des Gasmessschlauchs 6 im inneren Lumen L.e |
| 6.2 | exspiratorischer Verlauf des Gasmessschlauchs 6 im äußeren Lumen L.a |
| 6.3 | externer Verlauf des Gasmessschlauchs 6, verläuft außerhalb des Koaxialschlauchs 3 |
| 7 | T-Stück zwischen dem Koaxialschlauch 3 und dem medizinischen Gerät 1, gehört zum geräteseitigen Anschluss der Verbindungs-Anordnung |
| 8 | starres Endstück des äußeren Schlauchs des Koaxialschlauchs 3 |
| 9 | starres Endstück des inneren Schlauchs des Koaxialschlauchs 3, nimmt die Hülse 12.1 auf |
| 10 | Adapter gemäß der ersten Ausführungsform der Erfindung, hat die Ausgestaltung 10.1, 10.2 und 10.3 |
| 10.1 | Adapter gemäß der ersten Ausgestaltung der ersten Ausführungsform, umfasst eine erste, kleinere Hülse 10.1, eine zweite, größere Hülse 10.2 und eine koaxial angeordnete Röhre 13.1 im Inneren der beiden Hülsen 10.1, 10.2, bewirkt den inspiratorischen Verlauf 6.1 |
| 10.2 | Adapter gemäß der zweiten Ausgestaltung der ersten Ausführungsform, umfasst zusätzlich eine schräg angeordnete Verbindungsröhre 13.2, bewirkt den exspiratorischen Verlauf 6.2 |
| 10.3 | Adapter gemäß der zweiten Ausgestaltung der ersten Ausführungsform, umfasst zusätzlich eine schräg angeordnete längere Verbindungsröhre 13.3, bewirkt den externen Verlauf 6.3 |
| 11 | Adapter gemäß der zweiten Ausführungsform der Erfindung, hat die Ausgestaltungen 11.1, 11.2 und 11.3 |
| 11.1 | Adapter gemäß der ersten Ausgestaltung der zweiten Ausführungsform, umfasst die beiden Hülsen 10.1, 10.2 und die Führungsröhre 13.1, bewirkt den inspiratorischen Verlauf 6.1 |
| 11.2 | Adapter gemäß der zweiten Ausgestaltung der zweiten Ausführungsform, bewirkt den exspiratorischen Verlauf 6.2 |
| 11.3 | Adapter gemäß der zweiten Ausgestaltung der zweiten Ausführungsform, bewirkt den externen Verlauf 6.3 |
| 12.1 | erste, kleinere Hülse, von dem Endstück 9 aufgenommen |
| 12.2 | zweite, größere Hülse, schließt an das Endstück 9 an |
| 13.1 | koaxial angeordnete Röhre, hält den Gasmessschlauch 6 im inneren Lumen L.e (inspiratorischer Verlauf 6.1), hat ein patientenseitiges Ende an der Messposition X |
| 13.2 | schräg oder senkrecht angeordnete kürzere Verbindungsröhre, hält den Gasmessschlauch 6 im äußeren Lumen L.a (inspiratorischen Verlauf 6.2), in die Röhre 13.1 eingesetzt |
| 13.3 | schräg oder senkrecht angeordnete längere Verbindungsröhre, hält den Gasmessschlauch 6 in einer Position außerhalb des Koaxialschlauches 3 (externer Verlauf 6.3) , in die Röhre 13.1 eingesetzt |
| 13.4 | Segment der Röhre 13.1 hinter der kürzeren Verbindungsröhre 13.2 oder der längere Verbindungsröhre 13.3 |
| 14.1 | röhrenförmiges Führungselement, hält den Gasmessschlauch 6 |
| 14.2 | Öffnung im röhrenförmigen Führungselement 14.1 |
| 15.1 | kegelförmig sich verjüngender Innenraum in der ersten Hülse 12.1 |
| 20 | Mundstück, gehört zur patientenseitigen Kopplungseinheit, mit dem Verbindungsstück 5 verbindbar |
| d | Abstand zwischen der Messposition X und dem patientenseitigen Ende des Gasmessschlauchs 6 (nur erste Ausführungsform) |
| EA | vom Adapter 10, 11 gehaltener patientenseitiger Endabschnitt des Gasmessschlauchs 6 |
| En | patientenseitiges Ende des Y-Stücks 2 |
| L.a | das vom äußeren Schlauch gebildete äußere Lumen, vom Luftstrom S.a durchflossen |
| L.e | das vom inneren Schlauch gebildete innere Lumen, vom Luftstrom S.e durchflossen |
| M1, ... , M4 | nachteilhafte Messpositionen |
| P | Patient |
| S.a | Luftstrom beim Ausatmen (exspiratorischen Luftstrom), fließt durch das äußere Lumen L.a |
| S.e | Luftstrom beim Einatmen (inspiratorischen Luftstrom), fließt durch das innere Lumen L.e |
| Sp | Spülflow im Koaxialschlauch 3 |
| X | optimale Messposition; Position der patientenseitigen Spitze der Röhre 13.1 (erste Ausführungsform) bzw. des patientenseitigen Endes des Gasmessschlauchs 6 (zweite Ausführungsform) |

## Patentansprüche

1. Verbindungs-Anordnung zum Herstellen einer Fluidverbindung zwischen einem medizinischen Gerät (1) und einer patientenseitigen Kopplungseinheit (5, 20),
wobei die Verbindungs-Anordnung
- einen Versorgungsschlauch (3),
- ein patientenseitiges Verbindungsstück (2),
- einen biegsamen Fluidmessschlauch (6) und
- einen Adapter (10, 11) mit einer starren Adapter-Röhre (13.1, 14.1) und einer Adapter-Röhrenhalterung (12.1, 12.2)
umfasst,
wobei der Versorgungsschlauch (3)
- mit dem medizinischen Gerät (1) verbunden oder verbindbar ist und
- mit dem patientenseitigen Verbindungsstück (2) verbunden ist,
wobei der Versorgungsschlauch (3) und das patientenseitige Verbindungsstück (2) zusammen mindestens eine Fluidverbindung zwischen dem medizinischen Gerät (1) und der patientenseitigen Kopplungseinheit (5, 20) herstellen oder herzustellen vermögen,
wobei das patientenseitige Verbindungsstück (2) die Adapter-Röhrenhalterung (12.1, 12.2) hält,
wobei die Adapter-Röhre (13.1, 14.1) einen patientenseitigen Endabschnitt (EA) des Fluidmessschlauchs (6) hält,
wobei die Adapter-Röhrenhalterung (12.1, 12.2)
- die Adapter-Röhre (13.1, 14.1) vollständig oder wenigstens teilweise umgibt und
- den von der Adapter-Röhre (13.1, 14.1) gehaltenen Endabschnitt (EA) des Fluidmessschlauchs (6) in einer vorgegebenen Position relativ zum patientenseitigen Verbindungsstück (2) hält,
wobei die Verbindungs-Anordnung eine den Fluidmessschlauch (6) umfassende Mess-Fluidverbindung zwischen einem Fluidmessgerät (4) und einer vorgegebenen Fluidentnahmeposition (X), welche sich im Inneren des patientenseitigen Verbindungsstücks (2) befindet, herstellt oder herzustellen vermag und
wobei sich das patientenseitige Ende der Adapter-Röhre (13.1, 14.1) an der Fluidentnahmeposition (X) befindet.

2. Verbindungs-Anordnung nach Anspruch 1,
**dadurch gekennzeichnet, dass**
zwischen dem gehaltenen Endabschnitt (EA) des Fluidmessschlauchs (6) und der Fluidentnahmeposition (X) ein Abstand (d) auftritt,
wobei die Adapter-Röhre (13.1, 14.1) diesen Abstand (d) überbrückt und
wobei die hergestellte oder herstellbare Mess-Fluidverbindung zusätzlich zum Fluidmessschlauch (6) die Adapter-Röhre (13.1, 14.1) umfasst.

3. Verbindungs-Anordnung nach Anspruch 1,
**dadurch gekennzeichnet, dass**
sich an der Fluidentnahmeposition (X) zusätzlich zu dem patientenseitigen Ende der Adapter-Röhre (13.1, 14.1) das patientenseitige Ende des Fluidmessschlauchs (6) befindet.

4. Verbindungs-Anordnung nach einem der vorhergehenden Ansprüche, **dadurch gekennzeichnet, dass**
die Adapter-Röhre (13.1, 14.1) den gehaltenen Endabschnitt (EA) des Fluidmessschlauchs (6) fluiddicht umgibt oder
der gehaltene Endabschnitt (EA) des Fluidmessschlauchs (6) die Adapter-Röhre (13.1, 14.1) fluiddicht umgibt.

5. Verbindungs-Anordnung nach einem der vorhergehenden Ansprüche, **dadurch gekennzeichnet, dass**
ein Endstück (9) des Versorgungsschlauchs (3) mit der Adapter-Röhrenhalterung (12.1, 12.2) fluiddicht verbunden ist.

6. Verbindungs-Anordnung nach einem der vorhergehenden Ansprüche, **dadurch gekennzeichnet, dass**
die Adapter-Röhre (13.1, 14.1) vollständig oder wenigstens teilweise im Inneren des patientenseitigen Verbindungsstücks (2) angeordnet ist.

7. Verbindungs-Anordnung nach einem der vorhergehenden Ansprüche, **dadurch gekennzeichnet, dass**
der Innendurchmesser der Adapter-Röhre (13.1, 14.1) gegen eine Rückstellkraft vergrößerbar ist,
wobei der patientenseitige Endabschnitt (EA) des Fluidmessschlauchs (6) aufgrund der Rückstellkraft im Inneren der Adapter-Röhre (13.1, 14.1) eingeklemmt ist.

8. Verbindungs-Anordnung nach einem der vorhergehenden Ansprüche, **dadurch gekennzeichnet, dass**
die Fluidentnahmeposition (X) sich im Inneren des patientenseitigen Verbindungsstücks (2) und mit einem Abstand zu einem patientenseitigen Ende (En) des patientenseitigen Verbindungsstücks (2) befindet.

9. Verbindungs-Anordnung nach einem der vorhergehenden Ansprüche, **dadurch gekennzeichnet, dass**
das patientenseitige Verbindungsstück (2) eine Mittelachse aufweist, die parallel zu einer Fließrichtung von Fluid ist,
der Fluidmessschlauch (6) so positioniert ist, dass ein Abstand zwischen dem Fluidmessschlauch (6) und der Mittelachse des patientenseitigen Verbindungsstücks (2) auftritt und
die Adapter-Röhre (13.1, 14.1) in Fluidkommunikation mit einer Verbindungsröhre (13.2, 13.3) steht, die senkrecht oder schräg zur Mittelachse des patientenseitigen Verbindungsstücks (2) positioniert ist.

10. Verbindungs-Anordnung nach Anspruch 9,
**dadurch gekennzeichnet, dass**
zwischen den gehaltenen Endabschnitt (EA) des Fluidmessschlauchs (6) und der Fluidentnahmeposition (X) ein Abstand (d) auftritt,
wobei die Fluidverbindung zusätzlich zum Fluidmessschlauch (6) die Adapter-Röhre (13.1, 14.1) und die senkrecht oder schräg positionierte Verbindungsröhre (13.2, 13.3) umfasst.

11. Verbindungs-Anordnung nach einem der vorhergehenden Ansprüche, **dadurch gekennzeichnet, dass**
das patientenseitige Verbindungsstück (2) und der Adapter (10, 11) fest miteinander verbunden sind,
bevorzugt ein einziges Bauteil ausbilden.

12. Verbindungs-Anordnung nach einem der vorhergehenden Ansprüche, **dadurch gekennzeichnet, dass**
die Adapter-Röhre (13.1, 14.1) fest mit dem patientenseitigen Endabschnitt (EA) des Fluidmessschlauchs (6) verbunden ist,
insbesondere durch eine Klebeverbindung oder eine Schweißverbindung oder eine sonstige stoffschlüssige Verbindung.

13. Verbindungs-Anordnung nach einem der vorhergehenden Ansprüche, **dadurch gekennzeichnet, dass**
der Fluidmessschlauch (6) durch das Innere des Versorgungsschlauchs (3)
hindurch geführt ist.

14. Verbindungs-Anordnung nach Anspruch 13,
**dadurch gekennzeichnet, dass**
der Versorgungsschlauch (3) als ein Mehr-Lumen-Versorgungsschlauch ausgestaltet ist,
wobei der Mehr-Lumen-Versorgungsschlauch mindestens zwei Lumen (L.a, L.e) bereitstellt, welche fluiddicht voneinander getrennt sind,
wobei der Fluidmessschlauch (6) durch eines der bereitgestellten Lumen (L.a, L.e) geführt ist.

15. Verbindungs-System umfassend
- eine patientenseitige Kopplungseinheit (5, 20) und
- eine Verbindungs-Anordnung nach einem der vorhergehenden Ansprüche,
wobei die Verbindungs-Anordnung mit der patientenseitigen Kopplungseinheit (5, 20) verbunden ist.

16. Medizinisches System umfassend
- ein medizinisches Gerät (1),
- ein Fluidmessgerät (4) und
- ein Verbindungs-System gemäß Anspruch 15,
wobei das Fluidmessgerät (4) wenigstens zeitweise in einer Datenverbindung mit dem medizinischen Gerät (1) steht,
wobei der Versorgungsschlauch (3) mindestens eine Fluidverbindung zwischen dem medizinischen Gerät (1) und einen Patienten (P) herstellt und
wobei die Verbindungs-Anordnung eine den Fluidmessschlauch (6) umfassende Mess-Fluidverbindung zwischen dem Fluidmessgerät (4) und einer vorgegebenen Fluidentnahmeposition (X) herstellt.

17. Verfahren zur Herstellung einer Verbindungs-Anordnung nach Anspruch 13 oder Anspruch 14,
wobei das Verfahren die Schritte umfasst, dass
- der Fluidmessschlauch (6) dergestalt in den Versorgungsschlauch (3) hinein geschoben wird, dass nach den Einschieben ein patientenseitiger Endabschnitt (EA) des Fluidmessschlauchs (6) über den Versorgungsschlauch (3) übersteht,
- der Adapter (10, 11) so relativ zu dem Versorgungsschlauch (3) bewegt wird, dass bei der Bewegung der überstehende patientenseitige Endabschnitt des Fluidmessschlauchs (6) in die Adapter-Röhre (13.1, 14.1) eingeschoben wird,
- der Adapter (10, 11) mit dem patientenseitigen Verbindungsstück (2) verbunden wird und
- der Adapter (10, 11) mit dem Versorgungsschlauch (3) verbunden wird.

## Claims

1. Connection arrangement for establishing a fluid connection between a medical device (1) and a patient-side coupling unit (5, 20),
wherein the connection arrangement comprises
- a supply hose (3),
- a patient-side connection piece (2),
- a flexible fluid measurement hose (6) and
- an adapter (10, 11) comprising a rigid adapter tube (13.1, 14.1) and an adapter tube holder (12.1, 12.2),
wherein the supply hose (3)
- is connected or connectable to the medical device (1) and
- is connected to the patient-side connection piece (2),
wherein the supply hose (3) and the patient-side connection piece (2) together establish or are able to establish at least one fluid connection between the medical device (1) and the patient-side coupling unit (5, 20),
wherein the patient-side connection piece (2) holds the adapter tube holder (12.1, 12.2),
wherein the adapter tube (13.1, 14.1) holds a patient-side end section (EA) of the fluid measurement hose (6),
wherein the adapter tube holder (12.1, 12.2)
- completely or at least partially surrounds the adapter tube (13.1, 14.1) and
- holds, in a specified position relative to the patient-side connection piece (2), the end section (EA) of the fluid measurement hose (6) that is held by the adapter tube (13.1, 14.1),
wherein the connection arrangement establishes or is able to establish a measurement fluid connection, which comprises the fluid measurement hose (6), between a fluid measurement device (4) and a specified fluid removal position (X) located inside the patient-side connection piece (2) and
wherein the patient-side end of the adapter tube (13.1, 14.1) is located at the fluid removal position (X).

2. Connection arrangement according to Claim 1,
**characterized in that**
a distance (d) is found between the held end section (EA) of the fluid measurement hose (6) and the fluid removal position (X),
wherein the adapter tube (13.1, 14.1) bridges said distance (d) and
wherein the established or establishable measurement fluid connection comprises the adapter tube (13.1, 14.1) in addition to the fluid measurement hose (6).

3. Connection arrangement according to Claim 1,
**characterized in that**
the patient-side end of the fluid measurement hose (6) is located at the fluid removal position (X) in addition to the patient-side end of the adapter tube (13.1, 14.1).

4. Connection arrangement according to any of the preceding claims, **characterized in that**
the adapter tube (13.1, 14.1) surrounds the held end section (EA) of the fluid measurement hose (6) in a fluid-tight manner or
the held end section (EA) of the fluid measurement hose (6) surrounds the adapter tube (13.1, 14.1) in a fluid-tight manner.

5. Connection arrangement according to any of the preceding claims, **characterized in that**
an end piece (9) of the supply hose (3) is connected to the adapter tube holder (12.1, 12.2) in a fluid-tight manner.

6. Connection arrangement according to any of the preceding claims, **characterized in that**
the adapter tube (13.1, 14.1) is completely or at least partially arranged inside the patient-side connection piece (2).

7. Connection arrangement according to any of the preceding claims, **characterized in that**
the inner diameter of the adapter tube (13.1, 14.1) is enlargeable against a restoring force,
wherein the patient-side end section (EA) of the fluid measurement hose (6) is clamped inside the adapter tube (13.1, 14.1) owing to the restoring force.

8. Connection arrangement according to any of the preceding claims, **characterized in that**
the fluid removal position (X) is located inside the patient-side connection piece (2) and at a distance from a patient-side end (En) of the patient-side connection piece (2).

9. Connection arrangement according to any of the preceding claims, **characterized in that**
the patient-side connection piece (2) has a central axis parallel to a flow direction of fluid,
the fluid measurement hose (6) is positioned such that a distance is found between the fluid measurement hose (6) and the central axis of the patient-side connection piece (2) and
the adapter tube (13.1, 14.1) is in fluid communication with a connection tube (13.2, 13.3) positioned perpendicularly or diagonally to the central axis of the patient-side connection piece (2).

10. Connection arrangement according to Claim 9,
**characterized in that**
a distance (d) is found between the held end section (EA) of the fluid measurement hose (6) and the fluid removal position (X),
wherein the fluid connection comprises the adapter tube (13.1, 14.1) and the perpendicularly or diagonally positioned connection tube (13.2, 13.3) in addition to the fluid measurement hose (6).

11. Connection arrangement according to any of the preceding claims, **characterized in that**
the patient-side connection piece (2) and the adapter (10, 11) are fixedly connected to one another,
preferably form a single component.

12. Connection arrangement according to any of the preceding claims, **characterized in that**
the adapter tube (13.1, 14.1) is fixedly connected to the patient-side end section (EA) of the fluid measurement hose (6),
especially by an adhesive bond or a weld bond or some other integral bond.

13. Connection arrangement according to any of the preceding claims, **characterized in that**
the fluid measurement hose (6) is passed through the interior of the supply hose (3).

14. Connection arrangement according to Claim 13,
**characterized in that**
the supply hose (3) is designed as a multi-lumen supply hose,
wherein the multi-lumen supply hose provides at least two lumens (L.a, L.e) separated from one another in a fluid-tight manner,
wherein the fluid measurement hose (6) is passed through one of the provided lumens (L.a, L.e).

15. Connection system comprising
- a patient-side coupling unit (5, 20) and
- a connection arrangement according to any of the preceding claims,
wherein the connection arrangement is connected to the patient-side coupling unit (5, 20).

16. Medical system comprising
- a medical device (1),
- a fluid measurement device (4) and
- a connection system according to Claim 15,
wherein the fluid measurement device (4) has an at least temporary data connection with the medical device (1),
wherein the supply hose (3) establishes at least one fluid connection between the medical device (1) and a patient (P) and
wherein the connection arrangement establishes a measurement fluid connection, which comprises the fluid measurement hose (6), between the fluid measurement device (4) and a specified fluid removal position (X).

17. Method for producing a connection arrangement according to Claim 13 or Claim 14,
wherein the method comprises the steps of
- the fluid measurement hose (6) being pushed into the supply hose (3) such that, after push-in, a patient-side end section (EA) of the fluid measurement hose (6) projects beyond the supply hose (3),
- the adapter (10, 11) being moved relative to the supply hose (3) such that, upon movement, the projecting patient-side end section of the fluid measurement hose (6) is pushed into the adapter tube (13.1, 14.1),
- the adapter (10, 11) being connected to the patient-side connection piece (2) and
- the adapter (10, 11) being connected to the supply hose (3).

## Revendications

1. Ensemble de liaison destiné à établir une liaison fluidique entre un appareil médical (1) et une unité d'accouplement côté patient (5, 20), l'ensemble de liaison comprenant
- un tuyau d'alimentation (3),
- une pièce de liaison côté patient (2),
- un tuyau de mesure de fluide souple (6) et
- un adaptateur (10, 11) pourvu d'un tube adaptateur rigide (13.1, 14.1) et d'un support de tube adaptateur (12.1, 12.2),
le tuyau d'alimentation (3)
- étant relié ou pouvant être relié à l'appareil médical (1) et
- étant relié à la pièce de liaison côté patient (2),
le tuyau d'alimentation (3) et la pièce de liaison côté patient (2) établissant ou étant capables d'établir conjointement au moins une liaison fluidique entre l'appareil médical (1) et l'unité d'accouplement côté patient (5, 20),
la pièce de liaison côté patient (2) maintenant le support de tube adaptateur (12.1, 12.2),
le tube adaptateur (13.1, 14.1) maintenant une portion d'extrémité côté patient (EA) du tuyau de mesure de fluide (6), le support de tube adaptateur (12.1, 12.2)
- entourant complètement ou au moins partiellement le tube adaptateur (13.1, 14.1) et
- maintenant la portion d'extrémité (EA) du tuyau de mesure de fluide (6), maintenue par le tube adaptateur (13.1, 14.1), dans une position spécifiée par rapport à la pièce de liaison côté patient (2),
l'ensemble de liaison établissant ou étant capable d'établir une liaison de fluide de mesure, comprenant le tuyau de mesure de fluide (6), entre un appareil de mesure de fluide (4) et une position de prélèvement de fluide spécifiée (X) qui est située à l'intérieur de la pièce de liaison côté patient (2)
et
l'extrémité côté patient du tube adaptateur (13.1, 14.1) étant située à la position de prélèvement de fluide (X).

2. Ensemble de liaison selon la revendication 1,
**caractérisé en ce que**
une distance (d) apparaissant entre la portion d'extrémité maintenue (EA) du tuyau de mesure de fluide (6) et la position de prélèvement de fluide (X),
le tube adaptateur (13.1, 14.1) pontant cette distance (d) et
la liaison de fluide de mesure qui est établie ou peut être établie comprenant le tube adaptateur (13.1, 14.1) en plus du tuyau de mesure de fluide (6).

3. Ensemble de liaison selon la revendication 1,
**caractérisé en ce que**
l'extrémité côté patient du tuyau de mesure de fluide (6) est située à la position de prélèvement de fluide (X) en plus de l'extrémité côté patient du tube adaptateur (13.1, 14.1).

4. Ensemble de liaison selon l'une des revendications précédentes, **caractérisé en ce que**
le tube adaptateur (13.1, 14.1) entoure de manière fluidiquement étanche la portion d'extrémité maintenue (EA) du tuyau de mesure de fluide (6) ou
la portion d'extrémité maintenue (EA) du tuyau de mesure de fluide (6) entoure de manière fluidiquement étanche le tube adaptateur (13.1, 14.1).

5. Ensemble de liaison selon l'une des revendications précédentes, **caractérisé en ce que**
une pièce d'extrémité (9) du tuyau d'alimentation (3) est reliée de manière fluidiquement étanche au support de tube adaptateur (12.1, 12.2).

6. Ensemble de liaison selon l'une des revendications précédentes, **caractérisé en ce que**
le tube adaptateur (13.1, 14.1) est disposé complètement ou au moins partiellement à l'intérieur de la pièce de liaison côté patient (2).

7. Ensemble de liaison selon l'une des revendications précédentes, **caractérisé en ce que**
le diamètre intérieur du tube adaptateur (13.1, 14.1) peut être augmenté en s'opposant à une force de rappel,
la portion d'extrémité côté patient (EA) du tuyau de mesure de fluide (6) étant serrée à l'intérieur du tube adaptateur (13.1, 14.1) en raison de la force de rappel.

8. Ensemble de liaison selon l'une des revendications précédentes, **caractérisé en ce que**
la position de prélèvement de fluide (X) est située à l'intérieur de la pièce de liaison côté patient (2) et à distance d'une extrémité côté patient (En) de la pièce de liaison côté patient (2).

9. Ensemble de liaison selon l'une des revendications précédentes, **caractérisé en ce que**
la pièce de liaison côté patient (2) comporte un axe central qui est parallèle à une direction d'écoulement de fluide,
le tuyau de mesure de fluide (6) est positionné de sorte qu'une distance apparaisse entre le tuyau de mesure de fluide (6) et l'axe central de la pièce de liaison côté patient (2), et
le tube adaptateur (13.1, 14.1) est en communication fluidique avec un tube de liaison (13.2, 13.3) qui est positionné perpendiculairement ou obliquement à l'axe central de la pièce de liaison côté patient (2).

10. Ensemble de liaison selon la revendication 9,
**caractérisé en ce que**
une distance (d) apparaît entre la portion d'extrémité maintenue (EA) du tuyau de mesure de fluide (6) et la position de prélèvement de fluide (X),
la liaison de fluide comprenant, en plus du tuyau de mesure de fluide (6), le tube adaptateur (13.1, 14.1) et le tube de liaison (13.2, 13.3) positionné verticalement ou obliquement.

11. Ensemble de liaison selon l'une des revendications précédentes, **caractérisé en ce que**
la pièce de liaison côté patient (2) et l'adaptateur (10, 11) sont reliés l'un à l'autre de manière fixe, de préférence forment un seul composant.

12. Ensemble de liaison selon l'une des revendications précédentes, **caractérisé en ce que**
le tube adaptateur (13.1, 14.1) est relié de manière fixe à la portion d'extrémité côté patient (EA) du tuyau de mesure de fluide (6), notamment par une liaison adhésive ou une liaison soudée ou une autre liaison de matière.

13. Ensemble de liaison selon l'une des revendications précédentes, **caractérisé en ce que**
le tuyau de mesure de fluide (6) est guidé à travers l'intérieur du tuyau d'alimentation (3).

14. Ensemble de liaison selon la revendication 13,
**caractérisé en ce que**
le tuyau d'alimentation (3) est conçu sous la forme d'un tuyau d'alimentation à plusieurs lumières,
le tuyau d'alimentation à plusieurs lumières fournissant au moins deux lumières (La , Le) qui sont séparées l'une de l'autre de manière fluidiquement étanche,
le tuyau de mesure de fluide (6) étant guidé à travers l'une des lumières fournies (La, Le).

15. Système de liaison comprenant
- une unité d'accouplement côté patient (5, 20) et
- un ensemble de liaison selon l'une des revendications précédentes, l'ensemble de liaison étant relié à l'unité d'accouplement côté patient (5, 20).

16. Système médical comprenant
- un appareil médical (1),
- un appareil de mesure de fluide (4) et
- un système de liaison selon la revendication 15,
l'appareil de mesure de fluide (4) étant au moins temporairement en liaison de données avec l'appareil médical (1),
le tuyau d'alimentation (3) établissant au moins une liaison de fluide entre l'appareil médical (1) et un patient (P) et
l'ensemble de liaison établissant une liaison de fluide de mesure, comprenant le tuyau de mesure de fluide (6), entre l'appareil de mesure de fluide (4) et une position de prélèvement de fluide spécifiée (X).

17. Procédé de fabrication d'un ensemble de liaison selon la revendication 13 ou la revendication 14,
le procédé comprenant les étapes dans lesquelles
- le tuyau de mesure de fluide (6) est enfoncé dans le tuyau d'alimentation (3) de manière à ce que, après enfoncement, une portion d'extrémité côté patient (EA) du tuyau de mesure de fluide (6) fasse saillie du tuyau d'alimentation (3),
- l'adaptateur (10, 11) est déplacé par rapport au tuyau d'alimentation (3) de manière à ce que la portion d'extrémité côté patient saillante du tuyau de mesure de fluide (6) soit enfoncée dans le tube adaptateur (13.1, 14.1),
- l'adaptateur (10, 11) est relié à la pièce de liaison côté patient (2), et
- l'adaptateur (10, 11) est relié au tuyau d'alimentation (3).
